# EUROPÄISCHE PATENTANMELDUNG

(11) **EP 4 483 860 A1**
(43) Veröffentlichungstag der Anmeldung: **01.01.2025**
(21) Anmeldenummer: 23182868.2
(22) Anmeldetag: 30.06.2023
(51) Int. Cl.: A61K 6/62, A61K 6/887, B33Y 70/00, B33Y 80/00

(54) **RADIKALISCH POLYMERISIERBARE WERKSTOFFE ZUR HERSTELLUNG DENTALER FORMKÖRPER MIT HOHER BRUCHZÄHIGKEIT**

(71) Anmelder: Ivoclar Vivadent AG, 9494 Schaan (LI)
(72) Erfinder: Catel, Yohann, 9475 Sevelen (CH); Moszner, Norbert, 99441 Grossschwabhausen (DE); Fässler, Pascal, 8887 Mels (CH); Rist, Kai, 6800 Feldkirch (AT)
(74) Vertreter: Uexküll & Stolberg

(57) **Zusammenfassung**

Radikalisch polymerisierbare Zusammensetzung, die mindestens ein urethangruppenhaltiges (Meth)acrylatmonomer, mindestens einen Bruchzähigkeitsmodifikator und mindestens ein Übertragungsreagenz der allgemeinen Formel I enthält

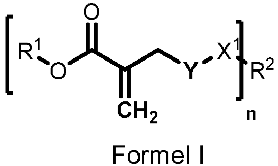

Die Zusammensetzung eignet sich insbesondere als Dentalwerkstoffe, beispielsweise zur Herstellung dentaler Formteile durch generative Verfahren, und zeichnet sich durch eine hohe Bruchzähigkeit aus.

## Beschreibung

Die vorliegende Erfindung betrifft radikalisch polymerisierbare Zusammensetzungen, die sich besonders als Dentalwerkstoffe zur Herstellung von dentalen Formkörpern, wie künstlichen Zähnen, Zahnprothesen, Inlays, Onlays, Splints, Kronen, Brücken, Verblendmaterialien und orthodontischen Vorrichtungen durch generative Verfahren eignen.

Klassische dentale Polymerisationssysteme bestehen meistens aus einer Mischung von Monomeren, Initiatorkomponenten, Stabilisatoren und Pigmenten (J. Viohl, K. Dermann, D. Quast, S. Venz, Die Chemie zahnärztlicher Füllungskunststoffe, Carl Hanser Verlag, München-Wien1986, 21-27). Dabei werden als Monomere für den Aufbau von Polymernetzwerken meistens Mischungen von Dimethacrylaten eingesetzt (vgl. A. Peutzfeldt, Resin composites in dentistry: The monomer systems, Eur. J. Oral. Sci. 105 (1997) 97-116; N. Moszner, T. Hirt, New Polymer-Chemical Developments in Clinical Dental Polymer Materials: Enamel-Dentin Adhesives and Restorative Composites, J. Polym. Sci. Part A: Polym. Chem. 50 (2012) 4369-4402). Beispiele hierfür sind die hochviskosen Dimethacrylate 2,2-Bis[4-(2-hydroxy-3-methacryloyloxypropyl)phenyl]propan (Bis-GMA) und 1,6-Bis-[2-methacryloyloxyethoxycarbonylamino]-2,4,4-tri-methylhexan (UDMA) sowie die als Verdünnermonomere genutzten niedrigviskosen Dimethacrylate Bismethacryloyloxymethyltricyclo[5.2.1.]decan (TCDMA), Decandiol-1,10-dimethacrylat (DsMA) und Triethylenglycoldimethacrylat (TEGDMA). Demgegenüber wird für Zahnprothesen hauptsächlich das monofunktionelle Monomer Methylmethacrylat (MMA) eingesetzt, das zwar niedrigviskos aber sehr flüchtig ist.

Je nach Anwendungsgebiet können weitere Additive zugesetzt werden. Zur Auslösung der radikalischen Polymerisation durch Licht werden Photoinitiatoren für den UVA-Bereich oder sichtbaren Bereich eingesetzt, die bei Bestrahlung mit UVA-Licht, meist mit einer Wellenlänge von 365 nm (Hg-Dampflampe) bis 395 nm (LED), oder Blaulicht (400-480 nm) Radikale bilden.

Prothesenmaterialien enthalten als eine wesentliche Komponente pulverförmiges Polymethylmethacrylat (PMMA), das mit MMA eine Paste bildet. Die Aushärtung erfolgt thermisch oder mit Redoxinitiatoren.

Ein wesentliches Problem bei der radikalischen Polymerisation von Methacrylaten ist der Polymerisationsschrumpf (ΔV_{P}), d.h. die bei der Polymerisation auftretende Volumenkontraktion der eingesetzten Methacrylatmonomere, was z.B. zu einer sehr nachteiligen Randspaltbildung bei Füllungskompositen führen kann oder bei Prothesenmaterialen die Dimensionsstabilität nachteilig beeinflusst. Der Polymerisationsschrumpf ΔV_{P} beträgt z.B. bei reinem MMA 21,0 Vol.-%.

Ein weiterer Nachteil von PMMA- oder Dimethacrylatpolymerisaten ist die große Sprödigkeit der Materialien. Die geringe Bruchzähigkeit ist eine inhärente Eigenschaft des amorphen PMMA-Glases und wird bei durch Dimethacrylatmischungen gebildeten Polymernetzwerken vor allem durch deren unregelmäßige Netzwerkstruktur verursacht. Außerdem zeigen Methacrylate einen unvollständigen Doppelbindungsumsatz, was im Fall von Monomethacrylaten einen merklichen Restmonomergehalt bedingen und so die Biokompatibilität der Polymerisate nachteilig beeinflussen kann.

Zur Herstellung dentaler Formkörper werden zunehmend additive Fertigungsverfahren (AM: additive manufacturing) eingesetzt. Diese werden auch als generative Fertigungsverfahren oder 3D-Druck bezeichnet und unter dem Begriff "Rapid Prototyping" (RP) zusammengefasst. Hierbei werden auf der Basis computergenerierter Konstruktionsdaten (CAD-Daten) dreidimensionale Modelle oder Bauteile schichtweise kontinuierlich hergestellt. Übliche Verfahren sind die Stereolithographie (SL), selektives Lasersintern (SLS), 3-D-Printing, Fused Deposition Modelling (FDM), Ink-Jet-Printing (IJP), 3D-Plotting, Multi-Jet Modelling (MJM), Solid Freeform Fabrication (SFF), Laminated Object Manufacturing (LOM), Laser Powder Forming (LPF). Mit diesen Verfahren lassen sich Modelle, Bau- oder Formteile auch in Kleinserie kostengünstig herstellen (A. Gebhardt, Generative Fertigungsverfahren, 3. Aufl., Carl Hanser Verlag, München 2007, 77ff.).

Bei der Stereolithographie wird ein Formteil auf der Basis von CAD-Daten schichtweise aus einem flüssigen und härtbaren Monomerharz aufgebaut, wobei das Härten der Schichten durch gesteuerte Belichtung und damit durch Photopolymerisation erfolgt (vgl. I. Gibson, D.W. Rosen, B. Stucker et al., "Additive Manufacturing Technologies", Band 238, Springer Verlag (2010)). Hierzu wird häufig ein UV-Laser als Lichtquelle verwendet. Beim DLP-Verfahren (**D**igital **L**ight **P**rocessing) verwendet man ein projizierbares Bild zur schichtenweise Aushärtung der Werkstoffe.

Für technische Anwendungen werden oft Photopolymersationsharze auf der Basis von Acrylaten oder Epoxiden eingesetzt, die sich durch eine hohe Reaktivität auszeichnen. Solche Monomersysteme sind wegen der mutagenen Eigenschaften vieler Acrylate und Epoxide für eine intraorale Härtung nicht geeignet. Hier werden meist Methacrylate eingesetzt, die eine deutlich bessere Biokompatibilität aufweisen aber im Vergleich zu Acrylaten eine wesentlich geringere Photopolymerisationsreaktivität haben.

Baumaterialien zur generativen Herstellung von dentalen Formkörpern müssen im Hinblick auf die Besonderheiten dieses Verfahrens unterschiedliche Anforderungen erfüllen. Sie sollen eine niedrige Viskosität und eine hohe Transparenz aufweisen und nach der Härtung gute mechanische Eigenschaften haben. Die hergestellten Formkörper müssen eine gute Biegefestigkeit und ein hohes E-Modul aufweisen und insbesondere eine gute Bruchzähigkeit haben.

Eine Verbesserung der Bruchzähigkeit lässt sich durch innere Weichmachung erzielen, z.B. durch die Zugabe von flexiblen Monomeren. Diese haben den Nachteil, dass sie die Biegefestigkeit und das E-Modul der Polymerisate deutlich verringern. Weiterhin können Polymerisationsharzen sog. Schlagzähmodifikatoren zugesetzt werden. Hierbei handelt es sich in der Regel um Polymerpartikel mit einer Kern-Schale-Struktur.

Die WO 2014/078537 A1 offenbart Harzmischungen zur Herstellung dentaler Formkörper durch 3D-Druckverfahren auf der Basis von mono- und multifunktionellen Methacrylaten, die Silicon-Acrylat-basierende Schlagzähmodifikatoren mit Kern-Schale-Struktur zur Verbesserung der Schlag- und Bruchzähigkeit erhalten.

Die US 2018/0000570 A1 betrifft Baumaterialien auf der Basis von mono- und multifunktionellen (Meth)acrylaten zur generativen Herstellung dentaler Bauteile. Die Baumaterialien enthalten Gummipartikel auf Silikon-Acryl-Basis mit Kern-Schale-Struktur als Schlagzähmodifikatoren und Oligomere, die durch Umsetzung von Trimethyl-1,6-diisocyanat, Bisphenol-A-propoxylat und 2-Hydroxyethylmethacrylat (HEMA) hergestellt werden. Die gehärteten Bauteile sollen gute mechanische und physikalische Eigenschaften sowie eine gute Bioverträglichkeit aufweisen.

Die US 10,299,896 B2 und die US 2019/0053883 A1 offenbaren durch generative Verfahren hergestellte dentale Bauteile, die mindestens zwei Schichten aus unterschiedlich zusammengesetzten Baumaterialien aufweisen. Dabei wird eine Schicht durch ein Material gebildet, das Oligomere, die durch Reaktion von Zwischenprodukten mit endständigen Isocyanatgruppen mit hydroxylbasierten Methacrylaten erhalten werden, eine polymerisierbare Acrylverbindung und einen Schlagzähmodifikator enthält. Mindestens eine weitere Schicht wird durch ein Material gebildet, das ein Urethanmonomer, ein Glycoldimethacrylat und Füllstoff enthält. Die Kombination von Materialen mit unterschiedlichen mechanischen und physikalischen Eigenschaften soll zur Anpassung der Bauteile an unterschiedliche Anforderungen vorteilhaft sein. Als Schlagzähmodifikatoren werden handelsübliche Polymere mit Kern-Schale-Struktur verwendet.

Die EP 3 564 282 A1 offenbart härtbare Zusammensetzungen für Hochtemperatur-Photopolymerisationsverfahren, die ein oligomeres Urethandimethacrylat als Glasübergangstemperaturmodifikator, ein (Poly-)Carbonat-(Poly-)-Urethan-Dimethacrylat als Zähigkeitsmodifikator und ggf. Core-Shell-Partikel enthalten. Sie sollen gute thermomechanische Eigenschaften und eine gute Bioverträglichkeit aufweisen und sich zur Herstellung kieferorthopädischer Vorrichtungen eignen.

Die Wirkungsweise von Schlagzähmodifikatoren mit Kern-Schale-Struktur beruht auf einer Wechselwirkung der Spitze eines sich bildenden Risses mit den Polymer-Partikeln. Die Partikel weisen einen relativ weichen Polymerkern und eine harte Polymerschale auf. Trifft eine Rissspitze auf einen solchen Partikel, kommt es zu einer Hohlraumbildung im Kern und zur Abtrennung der Polymerschale von der polymerisierten Harzmatrix sowie des Kerns von der Schale und damit zur Raumbildung (Kavitation) für plastische Deformationsflächen. Eine Verbesserung der Schlagzähigkeit ist nicht gleichbedeutend mit einer Verbesserung der Bruchzähigkeit. Kern-Schale-Partikel verleihen den Materialien eine relative gute Biegefestigkeit und ein relativ hohes E-Modul, sind aber in Monomermischungen nur schwer dispergierbar. Ein weiterer wesentlicher Nachteil für dentale Anwendungen ist, dass Kern-Schale-Polymere (CSP) die Transparenz der Materialien deutlich vermindern, was sich nachteilig auf den stereolithographischen Bauprozess auswirkt. Eine Verringerung der Transparenz reduziert zudem die Durchhärtungstiefe und kann so zu einer Verschlechterung der mechanischen Eigenschaften und einer Erhöhung des Restmonomergehalts führen.

Aus der EP 4 085 893 A1 sind radikalisch polymerisierbare Materialien zur Herstellung dentaler Formkörper durch generativen Verfahren bekannt, die zur Verbesserung der Bruchzähigkeit und Brucharbeit ein ABA- oder AB-Blockcopolymer enthalten. Die Materialien enthalten vorzugsweise außerdem 30 bis 70 Gew.-% mindestens eines monofunktionellen, radikalisch polymerisierbaren Monomers und mindestens ein radikalisch polymerisierbares Urethandi(meth)acrylattelechel mit einer zahlenmittleren Molmasse von 750 bis 2000 g/mol. Die Werkstoffe können zur Verbesserung der Bruch- und Schlagzähigkeit Kern-Schale-Polymere enthalten. Sie zeichnen sich durch einen relativ hohen Gehalt an monofunktionellen Monomeren aus.

Erfindungsgemäß wurde gefunden, dass eine deutliche Verbesserung der Bruchzähigkeit durch Blockcopolymere oder Kern-Schale-Polymere nur bei Polymernetzwerken mit einer geringeren Vernetzungsdichte erreicht werden kann. Eine geringe Vernetzungsdichte setzt die Zugabe erheblicher Anteile an monofunktionellen Monomeren voraus, was und zu einem erhöhten Restmonomergehalt und damit zu toxikologischen Problemen führen kann. Außerdem geht die Verwendung monofunktioneller Monomere mit einer Verschlechterung des E-Moduls einher.

Der Erfindung liegt die Aufgabe zugrunde, Materialien zur Herstellung dentaler Formkörper mit guter Bruchzähigkeit und hoher Brucharbeit zur Verfügung zu stellen. Die Materialien sollen eine niedrige Viskosität und eine gute Bioverträglichkeit haben und nach der Härtung gute mechanische Eigenschaften, insbesondere eine hohe Biegefestigkeit und ein hohes Biegemodul, nach Wasserlagerung haben. Die Materialien sollen außerdem schnell härtbar sein und einen hohen Monomerumsatz zeigen.

Die Aufgabe wird erfindungsgemäß durch radikalisch polymerisierbare Zusammensetzungen gelöst, die mindestens ein urethangruppenhaltiges (Meth)acrylatmonomer, mindestens einen Bruchzähigkeitsmodifikator und mindestens ein Übertragungsreagenz der allgemeinen Formel I enthalten: 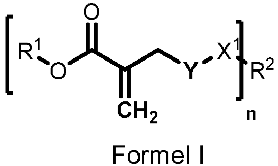 in der die Variablen die folgenden Bedeutungen haben:
- R¹: Wasserstoff, ein aliphatischer linearer oder verzweigter C₁-C₁₅-Alkyl-Rest, der durch ein oder mehrere, vorzugsweise 1 bis 3, O oder S unterbrochen und/oder durch eine oder mehrere, vorzugsweise 1, C₁-C₅-Alkylgruppen und/oder OH substituiert oder unsubstituiert sein kann, Benzyl oder Phenyl, die durch eine oder mehrere, vorzugsweise eine, C₁-C₃-Alkylgruppen substituiert oder unsubstituiert sein können;
- Y: S oder SO₂;
- X¹: entfällt oder ein aliphatischer linearer oder verzweigter C₁-C₁₅-Alkyl-Rest, der durch eine Estergruppe (-CO-O-), Urethangruppe (-O-CO-NH-), ein oder mehrere, vorzugsweise 1 bis 3, O oder S unterbrochen und/oder durch eine oder mehrere, vorzugsweise 1, C₁-C₅-Alkylgruppen und/oder OH substituiert oder unsubstituiert sein kann;
- R²: ein aliphatischer linearer oder verzweigter C₁-C₂₀-Alkyl-Rest, der durch eine Estergruppe (-CO-O-), Urethangruppe (-O-CO-NH-), ein oder mehrere, vorzugsweise 1 bis 3, O oder S unterbrochen und/oder durch 1 bis 3 OH Gruppen substituiert oder unsubstituiert sein kann, ein cycloaliphatischer C₅-C₂₀-Rest, ein aromatischer C₆-C₂₀-Rest oder ein Isocyanursäurerest, die jeweils durch eine oder mehrere, vorzugsweise eine, C₁-C₅-Alkylgruppen substituiert oder unsubstituiert sein können;
- n: 1, 2 oder 3.

Bevorzugt sind Verbindungen der Formel I bei denen die Variablen die folgenden Bedeutungen haben:
- R¹: aliphatischer linearer oder verzweigter C₁-C₁₀-Alkyl-Rest, der durch 1 bis 3 O oder S unterbrochen und/oder durch 1 bis 3 Methylgruppen substituiert oder unsubstituiert sein kann;
- Y: S oder SO₂;
- X¹: entfällt oder ein aliphatischer linearer oder verzweigter C₁-C₁₀-Alkyl-Rest, der durch eine Estergruppe (-CO-O-), Urethangruppe (-O-CO-NH-), 1 bis 3 O oder S unterbrochen und/oder durch 1 bis 3 Methylgruppen substituiert oder unsubstituiert sein kann;
- R²: ein aliphatischer linearer oder verzweigter C₁-C₁₆-Alkyl-Rest, der durch eine Estergruppe (-CO-O-), Urethangruppe (-O-CO-NH-), 1 bis 3 O oder S unterbrochen und/oder durch eine OH Gruppe substituiert sein kann, oder ein aromatischer C₆-C₂₀ Rest, der durch eine Methylgruppe substituiert oder unsubstituiert ist;
- n: 1 oder 2.

Besonders bevorzugt sind Verbindungen der Formel I bei denen die Variablen die folgenden Bedeutungen haben:
- R¹: ein aliphatischer linearer oder verzweigter C₁-C₆-Alkyl-Rest, der durch ein oder mehrere, vorzugsweise 1 bis 2, O oder S unterbrochen sein kann, vorzugsweise ein linearer C₁-C₃-Alkylrest;
- Y: S oder SO₂, vorzugsweise SO₂;
- X¹: entfällt;
- R²: ein aliphatischer linearer oder verzweigter C₁-C₁₂-Alkyl-Rest, der durch eine Estergruppe (-CO-O-), Urethangruppe (-O-CONH-), 1 bis 2 O oder S unterbrochen und/oder durch eine OH Gruppe substituiert oder unsubstituiert sein kann, oder ein aromatischer C₆-C₂₀ Rest, vorzugsweise eine methylsubstituierte Phenylgruppe;
- n: 1.

Alle hier gezeigten Formeln erstrecken sich nur auf solche Verbindungen, die mit der chemischen Valenzlehre vereinbar sind. Der Hinweis, dass ein Rest z.B. durch ein oder mehrere Sauerstoffatome unterbrochen ist, ist so zu verstehen, dass diese Atome jeweils in die Kohlenstoffkette des Restes eingeschoben werden. Diese Atome sind damit beidseitig durch C-Atome begrenzt und können nicht endständig sein. C₁-Reste können nicht verzweigt oder unterbrochen sein. Unter aromatischen Kohlenwasserstoffresten werden der üblichen Nomenklatur entsprechend auch solche Reste verstanden, die aromatische und nicht aromatische Gruppen enthalten. Ein bevorzugter aromatischer Rest ist beispielsweise die p-Tolylgruppe -Ph-CH₃.

Übertragungsreagenzien der Formel I sind zum Teil bekannt und können beispielsweise auf die in WO 2016/005534 A1 oder WO 2016/177677 A1 beschriebene Weise oder analog dazu hergestellt werden. Bevorzugte Beispiele für Verbindungen der Formel I sind:

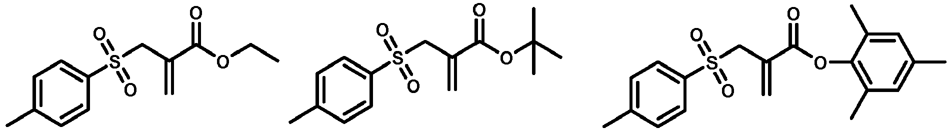

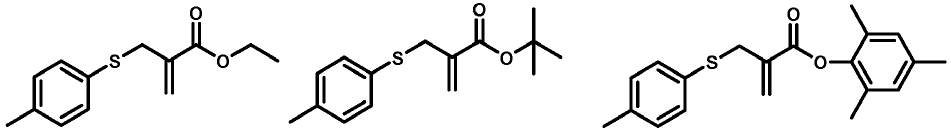

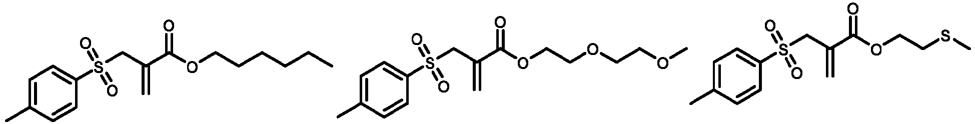

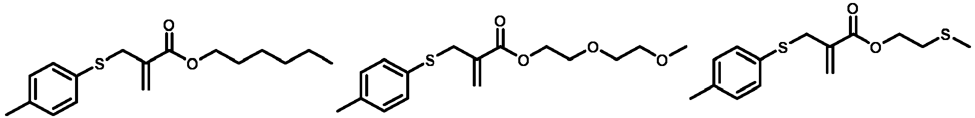

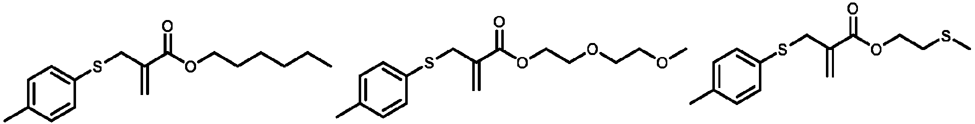

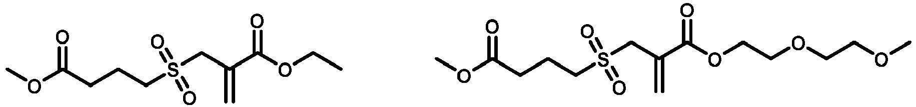

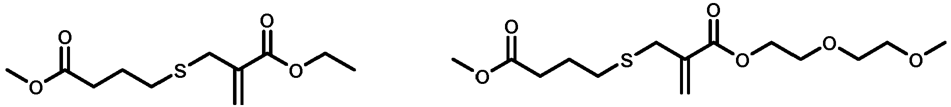

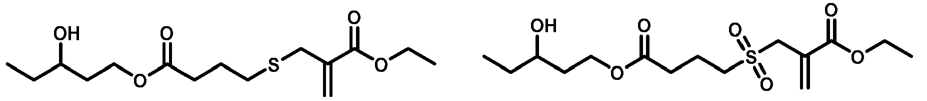

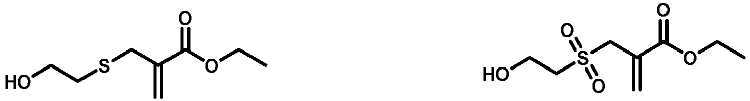

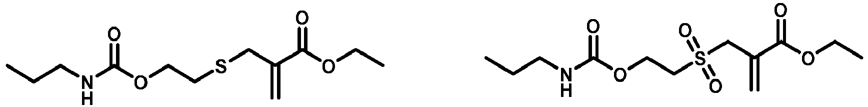

Als **urethangruppenhaltiges (Meth)acrylatmonomer** wird vorzugsweise ein Monomer oder eine Mischung von Monomeren verwendet, das oder die einen Urethangruppengehalt von 0,01 bis 5 mmol/g, vorzugsweise 0,1 bis 4,5 mmol/g und besonders bevorzugt 0,5 bis 4,0 mmol/g aufweist/aufweisen. Besonders bevorzugt sind Urethandi(meth)acrylate, ganz besonders Urethandi(meth)acrylate mit einer Molmasse von kleiner 2000 g/mol, und am meisten bevorzugt Urethandi(meth)acrylate mit einer Molmasse von kleiner 2000 g/mol, die 1 bis 4 Urethangruppen enthalten.

Erfindungsgemäß bevorzugte urethangruppenhaltige (Meth)acrylatmonomere sind erhältlich durch:
**A)** Reaktion von 2 Mol 2-Isocyanatoethyl(meth)acrylat (R⁴: H oder CH₃) mit einem Mol an α,ω-Alkandiolen, cycloaliphatischen, tricyclischen oder aromatischen Diolen sowie ethoxylierten oder propoxylierten aromatischen Diolen (R³: C₁-C₂₀ aliphatisches, cycloaliphatisches oder tricyclisches Alkylen oder C₆-C₁₄-Arylen bzw. alkoxyliertes C₆-C₁₄-Arylen): 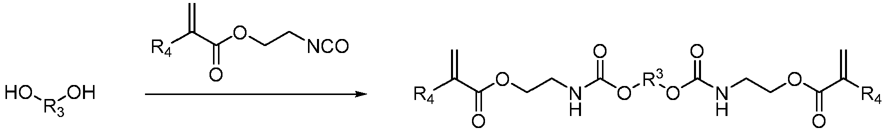
**B)** Reaktion von 1 Mol an einem oder mehreren Diisocyanaten (R⁵: C₁-C₁₂ aliphatisches, cycloaliphatisches Alkylen oder C₆-C₁₄-Arylen, das Alkylsubstituenten tragen kann) mit 2 Mol 2-Hydroxyalkyl(meth)acrylat, Poly(ethylenglycol)- oder Poly(propylenglycol)mono(meth)acrylat sowie Mischungen davon (R⁶: H oder CH_{3;} R⁷: lineares oder verzweigtes C₁ -C₄₀-Alkylen, wobei die Alkylenkette durch ein oder mehrere O-Atome unterbrochen sein kann): 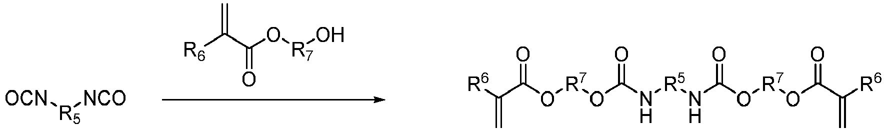
**C)** Reaktion von 1 Mol an ethoxyliertem oder propoxyliertem Bisphenol-A, Decandiol oder Dodecandiol oder anderen α,ω-Alkandiolen, cycloaliphatischen, tricyclischen oder aromatischen Diolen bzw. ethoxy- bzw. propoxylierten aromatischen Diolen (R³: C₁-C₂₀ aliphatisches, cycloaliphatisches oder tricyclisches Alkylen oder C₆-C₁₄-Arylen bzw. alkoxyliertes C₆-C₁₄-Arylen) mit 2 Mol Isophorondiisocyanat (IPDI) und anschließender Umsetzung mit 2 Mol 2-Hydroxyethyl(meth)acrylat oder Hydroxypropyl(meth)acrylat (R⁶: H oder CHs; R⁷: lineares oder verzweigtes C₁-C₄₀-Alkylen, wobei die Alkylenkette durch ein oder mehrere O-Atome unterbrochen sein kann): 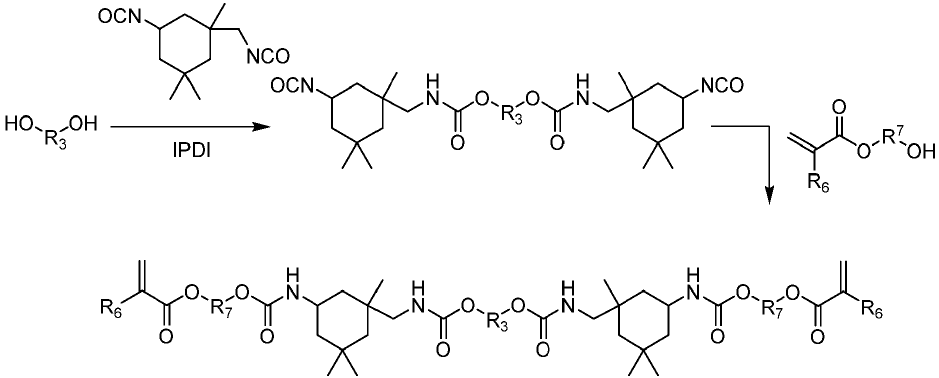
**D)** Reaktion von 1 Mol 2-Isocyanatoethyl(meth)acrylat mit 1 Mol 2-Hydroxyalkyl-(meth)acrylat, Poly(ethylenglycol)- oder Poly(propylenglycol)mono(meth)acrylat (R⁷: lineares oder verzweigtes C₁-C₄₀-Alkylen, wobei die Alkylenkette durch ein oder mehrere O-Atome unterbrochen sein kann; R⁴, R⁶: H oder CH₃):

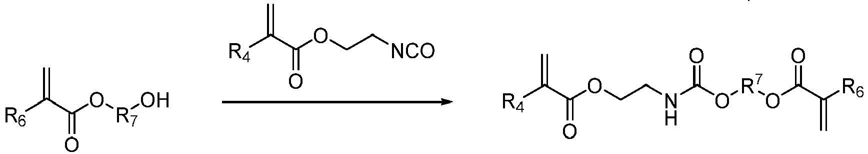

Erfindungsgemäß besonders bevorzugt sind Urethandi(meth)acrylate mit eine Molmasse kleiner 750 g/mol, die gemäß Syntheseweg A erhältlich sind durch:
Reaktion von 2 Mol 2-Isocyanatoethyl(meth)acrylat mit einem Mol an α,ω-Alkandiolen (Ethylenglycol, Propylenglcol, Butan-1,4-diol, Pentan-1,5-diol, Neopentylglycol, Hexan-1,6-diol, 2,2,4-Timethylhexandiol, 2,2,4-Trimethyl-1,3-pentandiol, Heptan-1,7-diol, Octan-1,8-diol, Nonan-1,9-diol, Decan-1,10-diol, Undecan-1,11-diol oder Dodecan-1,12-diol), C₆-C₁₈-Alkandiolen, die 1 bis 4 O- oder S-Atome in der Kohlenstoffkette enthalten können, cycloaliphatischen Diolen (2-Methyl-1,4-cyclopentandiol, 1,3- oder 1,4-Cyclohexandiol, Cyclooctandiol, Methylcycloheptandiol, 2,5-Dimethyl-1,4-cyclohexandiol, 3,3,5-Trimethylcyclopentan-1,2-diol, 2,2,4,4-Tetramethyl-1,3-cyclobutandiol), tricyclischen (Tricyclodecandimethanol: Tricyclo[5.2.1.0^{2,6}]decandimethanol) oder aromatischen, ethoxy- oder propoxylierten Diolen mit im Mittel 2, 3 oder 4 Ethoxy- oder Propoxygruppen (Bisphenol-A, Bisphenol-B, Bisphenol-E, Bisphenol-F, Bisphenol-S, Hydrochinon, Resorcin, 2,6-Dihydroxynaphthalin oder 4,4'-Dihydroxybiphenyl).

Ebenso besonders bevorzugt sind Urethandi(meth)acrylate mit eine Molmasse kleiner 750 g/mol, die gemäß Syntheseweg B erhältlich sind durch:
Reaktion von 1 Mol an Hexamethylen-1,6-diisocyanat (HMDI), 2,2,4-Trimethylhexamethylen-1,6-diisocyanat (TMDI), Isophorondiisocyanat (IPDA), Diphenylmethan-4,4'-diisocyanat 4,4'-MDI), hydriertem Diphenylmethan-4,4'-diisocyanat (H₁₂-MDI: 4,4'-Methylen-bis-(cyclohexylisocyanat) oder Tetramethylxylylendiisocyanat (TMXDI: 1,3-Bis(2-isocyanatopropan-2-yl)benzol) oder deren Mischungen mit 2 Mol 2-Hydroxyethyl(meth)acrylat, Hydroxypropyl(meth)acrylat, Poly(ethylenglycol)-200- mono-(meth)acrylat oder Poly(propylenglycol)-200-mono(meth)acrylat.

Weitere bevorzugte Urethandi(meth)acrylate sind die in EP 4 085 893 A1 offenbarten Urethandi(meth)acrylat-Telechele mit einer Molmasse von 800 bis 2000 g/mol. Diese sind durch Umsetzung von Diisocyanaten mit Diolen (HO-DA-OH) und anschließender Reaktion der α,ω-isocyanat-funktionalisierten Urethantelechele mit 2-Hydroxyethylmethacrylat (HEMA) oder Hydroxypropylmethacrylat (HPMA) erhältlich. DA steht für einen aromatischen oder aliphatischen Kohlenwasserstoffrest mit 6 bis 33 Kohlenstoffatomen, vorzugsweise einen divalenten polycyclischen Kohlenwasserstoffrest, insbesondere einen o-Diphenyl-, p-Diphenyl- oder Bisphenol A-Rest, oder eine verzweigte oder vorzugsweise lineare C₂-C₁₈-Alkylengruppe. Die Kohlenwasserstoffreste können ein oder mehrere O-Atome und/oder S-Atome enthalten, wobei O-Atome bevorzugt sind.

Bevorzugte Diole der Formel HO-DA-OH sind ethoxyliertes- oder propoxyliertes Bisphenol-A, o-Diphenyl oder p-Diphenyl mit 2 bis 6 Ethoxy- oder Propoxy-Gruppen sowie C₂-C₁₈-Alkandiole, die 1 bis 4 O- oder S-Atome in der Kohlenstoffkette enthalten können. Besonders bevorzugte Diole sind ethoxyliertes- oder propoxyliertes Bisphenol-A mit 2, 3 oder 4 Ethoxy- oder Propoxygruppen, Hexan-1,6-diol, Octan-1,8-diol, Nonan-1,9-diol, Decan-1,10-diol oder Dodecan-1,12-diol, Tetra- oder Pentaethylenglycol. Ganz besonders bevorzugt sind ethoxyliertes- oder propoxyliertes Bisphenol-A mit 2 oder 3 Ethoxy- oder Propoxygruppen, Decan-, Undecan- oder Dodecandiol sowie cyclische oder polycyclische aliphatische Diole, insbesondere Cyclohexandiol, Norbornandiol, Tricyclodecandiol und Tricyclodecandimethanol (Octahydro-4,7-methano-1H-indendimethanol).

Bevorzugte Diisocyanate sind Hexamethylen-1,6-diisocyanat (HMDI), 2,2,4-Trimethyl-hexamethylen-1,6-diisocyanat (TMDI), 1-Isocyanato-3-isocyanatomethyl-3,5,5-trimethylcyclohexan (Isophorondiisocyanat, IPDI), m-Tetramethylxylylendiisocyanat, (1,3-Bis(2-isocyanato-2-propyl)benzol, TMXDI), Toluol-2,4-diisocyanat (TDI), Diphenylmethan-4,4'-diisocyanat (MDI) und 1-Isocyanato-4-[(4-isocyanatocyclohexyl)methyl]cyclohexan (H₁₂MDI), wobei IPDI besonders bevorzugt ist.

Besonders bevorzugt sind Telechele gemäß der allgemeinen Formel (II) 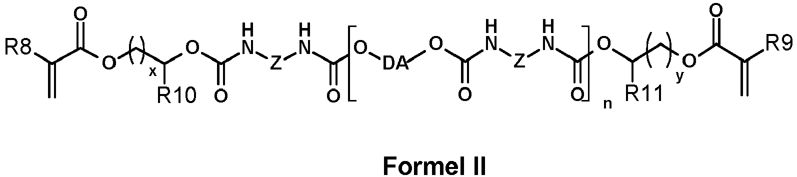 in der die Variablen die folgenden Bedeutungen haben:
- R⁸, R⁹: unabhängig voneinander jeweils H oder Methyl, vorzugsweise Methyl,
- R¹⁰, R¹¹: unabhängig voneinander jeweils H oder Methyl, vorzugsweise Methyl,
- x, y: unabhängig voneinander jeweils eine ganze Zahl von 1 bis 11, vorzugsweise 1 bis 5,
- n: 1, 2 oder 3, vorzugsweise 1,
- DA: ein Strukturelement, das sich aus den Diolen HO-DA-OH durch Abspaltung der Wasserstoffatome der beiden Hydroxylgruppen ableitet,
Z 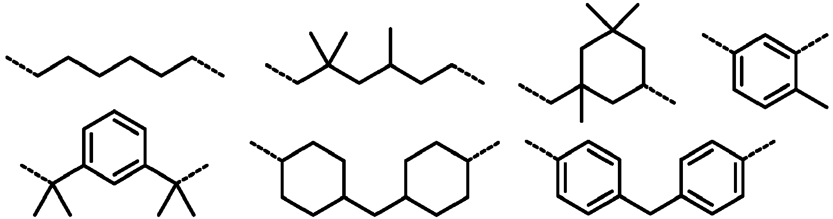 vorzugsweise :

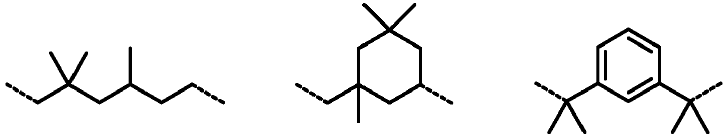

Die Urethandi(meth)acrylat-Telechele tragen zwei radikalisch polymerisierbare (Meth)acrylatgruppen und eigenen sich als Vernetzer. Sie zeichnen sich durch eine gute radikalische Polymerisationsfähigkeit aus und ergeben aufgrund ihrer relativ hohen Molmasse Polymerisate mit einer geringeren Netzwerkdichte und geringem Polymerisationsschrumpf.

Ganz besonders bevorzugte Urethandi(meth)acrylate sind UDMA (1,6-Bis-[2-methacryloyloxyethoxycarbonylamino]-2,4,4-trimethylhexan = Additionsprodukt von 1 mol 2,2,4-Trimethylhexamethylen-1,6-diisocyanat und 2 mol 2-Hydroxyethylmethacrylat), V-380 (Additionsprodukt von 1 mol α,α,α`,α`-Tetramethyl-m-xylylendiisocyanat und einer Mischung von 1,4 mol 2-Hydroxyethylmethacrylat und 0,6 mol 2-Hydroxypropylmethacrylat) und V-818 (Additionsprodukt von 1 mol Tricyclo[5.2.1.0^{2,6}]decandimethanol und 2 mol 2-Isocyanatoethylmethacrylat). UDMA weist einen Urethangruppengehalt von 4,235 mmol/g auf.

Die erfindungsgemäßen Zusammensetzungen enthalten mindestens einen **Bruchzähigkeitsmodifikator,** vorzugsweise mindestens ein Blockcopolymer.

Unter Blockcopolymeren werden Makromoleküle verstanden, die aus zwei oder mehr kovalent miteinander verbundenen Co- oder vorzugsweise Homopolymerblöcken bestehen. Erfindungsgemäß bevorzugte Blockcopolymere sind AB-Diblock- und insbesondere ABA-Triblockcopolymere.

Der A-Block ist ein Polymerisat, vorzugsweise ein Oligomer, das aus einem oder mehreren der folgenden Monomere aufgebaut ist: cyclische, aliphatische Ester oder Ether, Arylenoxid, Alkylenoxid, radikalisch polymerisierbare Monomere, beispielsweise α,β-ungesättigte Säuren und α,β-ungesättigte Säureester. Vorzugsweise ist der Block A ein Poly(meth)acrylat-, Polylacton-, Polyphenylenoxid- oder Polyalkylenoxid-Oligomer. Ganz besonders bevorzugt ist der Block A ein Polymerisat von Caprolacton, 2,6-Dialkyl-1,4-phenylenoxid und insbesondere von 2,6-Dimethyl-1,4-phenylenoxid, Ethylenoxid, Propylenoxid oder (Meth)acrylaten. Der A-Block ist damit vorzugsweise ein Polycaprolacton- (PCL), Poly(2,6-dimethyl-1,4-phenylenoxid)-, Poly(ethylenoxid)-, Poly(propylenoxid)- oder Poly(meth)acrylat-Oligomer.

Der B-Block ist vorzugsweise ein Polysiloxan- und/oder ein Polyvinyl- und/oder ein Polyalken- und/oder ein Polydien-Oligomer bzw. ein hydriertes Polydien-Oligomer. Besonders bevorzugt ist der B-Block ein Polydien-Oligomer bzw. ein hydriertes Polydien-Oligomer, Polyvinyalkanoat-Oligomer oder ein Polysiloxan-Oligomer gemäß der Formel:

-O-(SiR¹²₂-O)ₚ-

in der
- R¹²: eine lineare C₁-C₂₀-Alkyl-, verzweigte C₃-C₁₂-Alkyl- oder C₆-C₂₀-Arylgruppe ist, wobei die einzelnen R₁₂-Reste gleich oder verschieden sein können, und
- p: eine Zahl von 3 bis 100, bevorzugt eine Zahl von 10 bis 50 ist.

Ganz besonders bevorzugt ist der B-Block ein Polymerisat von Dimethylchlorsilan, Cyclotri- oder Cyclotetradimethoxysilan, Isopren, Vinylacetat, Isobuten, cis-Butadien oder Ethylen. Der B-Block ist damit vorzugsweise ein Poly(dimethylsiloxan)- (PDMS), hydriertes Poly(isopren)-, Poly(vinylacetat)-, hydriertes Poly(isobuten)-, hydriertes cis-Poly(butadien)- oder Poly(ethylen)-Oligomer.

Die B-Blöcke zeichnen sich durch eine relativ hohe Flexibilität aus. Unter flexiblen Blöcken werden Blöcke verstanden, die aus Monomeren gebildet werden, deren Homopolymere eine Glasübergangstemperatur T_{G} unter 50°C, vorzugsweise unter 0°C und ganz besonders bevorzugt im Bereich von -30 bis -110°C haben. Blockcopolymere mit flexiblen Blöcken verbessern die Bruchzähigkeit, beinträchtigen aber die Biegefestigkeit und das E-Modul der Polymerisate deutlich weniger als innere Weichmacher.

Bevorzugt sind ABA- und AB-Blockcopolymere bei denen der oder die A-Blöcke vorzugsweise jeweils ein oligomerer Polycaprolacton-, Poly(2,6-dimethyl-1,4-phenylen-oxid)-, Poly(ethylenoxid)-, Poly(propylenoxid)-, Poly(meth)acrylat- oder Poly(meth)acrylat-Styrol-Copolymer-Baustein sind und der B-Block vorzugsweise ein flexibler Poly-(dimethylsiloxan)-, Poly(isopren)- oder hydriertes Poly(isopren)-, Poly(vinylacetat)-, Poly(isobuten)-, cis-Poly(butadien)- oder hydriertes cis-Poly(butadien)- oder ein Poly(ethylen)-Baustein ist.

Erfindungsgemäß besonders bevorzugt sind ABA-Blockcopolymere in denen die A-Blöcke jeweils ein oligomerer Polycaprolacton-, Poly(meth)acrylat- oder Poly(meth)acrylat-Styrol-Copolymer-Baustein sind und der B-Block ein flexibler Poly(dimethylsiloxan)-, hydriertes Poly(isopren)- oder hydriertes cis-Poly(butadien)-Baustein ist.

Ganz besonders bevorzugt sind Polyester-Polysiloxan-Blockcopolymere gemäß der folgenden allgemeinen Formel:

(PCL)_{q}-b-(PDMS)ᵣ-b-(PCL)_{q}

in der
- q: jeweils eine Zahl von 5 bis 40, bevorzugt 10 bis 20, ist und
- r: eine Zahl von 10 bis 100, bevorzugt 30 bis 60, ist.
(PCL)_{q} steht für Polycaprolacton, das aus q Caprolactonmonomeren aufgebaut ist, und (PDMS)r für Poly(dimethylsiloxan), das aus r Dimethylsiloxanmonomeren aufgebaut ist. Der Buchstabe b steht für *block.*

Weiter bevorzugt sind Poly(meth)acrylat-Polysiloxan-Blockcopolymere, die als A-Block einen Polymethylmethacrylat-Rest und als B-Block einen Polysiloxan-Rest enthalten, wobei der Polysiloxan-Rest vorzugsweise wie oben definiert und ganz besonders bevorzugt ein Poly(dimethylsiloxan)-Rest ist.

Besonders bevorzugt sind außerdem die ABA-Triblockcopolymere PCL-b-PDMS-b-PCL und PMMA-b-PDMS-b-PMMA mit einem Molverhältnis A : B von 0,1 bis 5 und mit einer Molmasse von bevorzugt 3 bis 25 kDa, besonders bevorzugt 4 bis 20 kDa und ganz besonders bevorzugt 5 bis 10 kDa. Ein bevorzugtes Blockcopolymer ist PCL-b-PDMS-b-PCL, wobei die PDMS-Blöcke eine Molmasse von ca. 3200 g/mol und die PCL-Blöcke eine Molmasse von jeweils ca. 1600 g/mol aufweisen. PCL steht für Polycaprolacton, PDMS für Poly(dimethylsiloxan) und PMMA für Polymethylmethacrylat.

Blockcopolymere lassen sich mit den bekannten Methoden der lebenden bzw. kontrollierten Polymerisation herstellen, z.B. durch radikalische oder ionische (anionische und kationische) Polymerisation, und auch durch metallkatalysierter Ringöffnungspolymerisation von cyclischen Monomeren, wie z.B. Caprolacton, wobei die kontrollierte radikalische Polymerisation, die lebende anionische Polymerisation und Ringöffnungspolymerisation bevorzugt sind. Blockcopolymere können aber auch durch die Verknüpfung von Endgruppen von Homopolymeren erhalten werden. Die erfindungsgemäß verwendeten Blockcopolymere können als Di- und Triblockcopolymere vorliegen.

AB-Blockcopolymere lassen sich beispielsweise herstellen, indem ein A-Block mit endständiger OH-Gruppe durch Veresterung mit einem B-Block verknüpft wird, der eine COOH-Gruppe aufweist. Endgruppenfunktionalisierte Homopolymerblöcke lassen sich relativ einfach mit den Methoden der geregelten radikalischen Polymerisation oder durch Endcapping bei der anionischen Polymerisation herstellen. Beispielsweise wird das Monomer A anionisch polymerisiert und durch Endcapping eine OH-Gruppe eingeführt. Die OH-Endgruppe lässt sich dann z.B. mit α-Bromisobuttersäure verestern. Die dabei erhaltene Brom-Endgruppe fungiert dann als Startzentrum für die Bildung des B-Blocks durch ATRP (Atom Transfer Radical Polymerization) von Monomer B, initiiert durch Metallkomplexe beispielsweise von Cu(I), Ru(l) oder Fe(II).

Triblockcopolymere lassen sich auf analoge Weise herstellen. Beispielsweise wird durch anionische Polymerisation von Monomer B über einen Dianionen-Mechanismus ein B-Block hergestellt. Der gebildete B-Mittelblock trägt beidseitig jeweils eine Anion-Endgruppe, die die anionische Polymerisation von Monomer A unter Bildung der beiden A-Blocke initiiert (Methode 1). Die Veresterung eines telechelen B-Blocks, der an beiden Enden jeweils eine geeignete funktionelle Gruppe, z.B. eine OH-Gruppe trägt, mit zwei A-Blöcken, die nur einseitig z.B. mit einer COOH-Gruppe, funktionalisiert sind, ergibt ABA-Triblockcopolymere (Methode 2). Schließlich lassen sich OH-telechele Homopolymere von Monomer B mit α-Bromisobuttersäure verestern. Die beiden so gebildeten Brom-Endgruppen im Homopolymerblock B lassen sich dann als Startzentrum für die Bildung der beiden A-Blöcke durch ATRP nutzen (Methode 3).

Bei der Synthese der Blockcopolymeren lassen sich auch end- oder seitenständig polymerisationsfähige (Meth)acrylatgruppen einführen. Diese bewirken eine bessere Einbindung der Blockcopolymere in die gebildeten Polymernetzwerke durch radikalische Copolymerisation der (Meth)acrylatgruppen.

Die Monomere werden vorzugsweise so gewählt, dass die A-Blöcke mit der Harz-matrix, d.h. der Mischung der Bestandteile (a) bis (d), mischbar und der B-Block nicht mit der Harzmatrix mischbar ist. Die Mischbarkeit wird hier im Sinne der Thermodynamik in Bezug auf die Einphasigkeit verstanden. Danach wird unter einem mischbaren Polymerblock ein Polymerblock aus einem Monomer verstanden, dessen Homopolymer in der Harz-Matrix löslich ist, so dass die Mischung eine Transparenz von mindestens 95 % aufweist. Ist die Mischung dagegen trüb oder opak, d.h. ist die Transparenz deutlich kleiner als 95 %, so ist das Homopolymer und damit der entsprechende Polymerblock nicht mit der Harz-Matrix mischbar. Die Transparenz wird mit einem Spektrophotometer an 1 mm dicken, auf Hochglanz polierten Prüfkörpern in Transmission (D65) gemäß der Norm ISO 10526:1999 gemessen, z.B. mit einem Konika-Minolta Spektrophotometer des Typs CM-5.

Die erfindungsgemäßen Zusammensetzungen können als Bruchzähigkeitsmodifikator alternativ oder vorzugsweise zusätzlich zu den Blockcopolymeren ein oder mehrere Polymerpartikel mit Kern-Schale-Struktur enthalten. Bevorzugt sind Polymerpartikel, die eine weichen Kern, vorzugsweise aus Polybutadien, hydriertem Polybutadien, Polyisopren, hydriertem Polyisopren, Polybutylacrylat, MMA-Butadien-Styrol-Copolymeren (MBS) oder Polydimethylsiloxan, und eine harte Schale, vorzugsweise aus PMMA oder einem MMA-Styrol-Copolymer aufweisen.

Unter weichen oder flexiblen Polymeren werden Polymere mit einer Glasübergangstemperatur T_{G} unter 50°C, vorzugsweise unter 0°C und ganz besonders bevorzugt im Bereich von -30 bis -110°C verstanden. Ein bevorzugtes konkretes Beispiel ist PDMS mit einer T_{G} von ca. -110°C. Unter harten Polymeren werden Polymere mit einer Glasübergangstemperatur über 50°C und bevorzugt über 80°C verstanden. Ein bevorzugtes konkretes Beispiel ist PMMA mit einer Tcvon 105-120°C.

Die bruchzähigkeitsmodifizierende Wirkung der CSP-Partikeln in radikalischen Di(meth)acrylat-Polymernetzwerken hängt vor allem von der Art der CSP-Partikel, der Partikelgröße, der Vernetzungsdichte und dem Gewichtsverhältnis von Kern zu Schale ab, das vorzugweise in einem Bereich von 1:1 bis 200:1 liegt. Die Vernetzungsdichte wird maßgeblich durch den Anteil vernetzender Monomere im Partikelkern bestimmt. Dieser liegt vorzugsweise in einem Bereich von 1 bis 10 Gew.-%, bezogen auf die Masse des Kerns. Erfindungsgemäß sind Partikel mit einer Teilchengröße von 0,20 bis 50,0 µm bevorzugt. Bei der Einarbeitung der CSP-Partikel in den Dentalwerkstoff ist auf eine gute Dispergierung zu achten.

Der oder die Bruchzähigkeitsmodifikatoren werden vorzugsweise in einer Menge von 1 bis 10 Gew.-%, besonders bevorzugt in einer Menge von 2 bis 8 Gew.-% und ganz besonders bevorzugt 3 bis 6 Gew.-% eingesetzt, bezogen auf das Gesamtgewicht des Dentalwerkstoffs.

Kern-Schale-Polymeren haben den Nachteil, dass sie die Transparenz der Zusammensetzungen beeinträchtigen können, was sich bei der Photopolymerisation negativ auf die Durchhärtungstiefe und bei dentalen Formkörpern zusätzlich negativ auf die Ästhetik auswirkt. Erfindungsgemäß sind daher Werkstoffe bevorzugt, die maximal 3 Gew.-% und besonders bevorzugt keine Kern- Schale-Partikel enthalten.

Wenn nicht anders angegeben, handelt es sich hierin bei der Molmasse von Oligomeren und Polymeren um die zahlenmittlere Molmasse Mₙ, die mittels Gelpermeationschromatographie (GPC) bestimmt wird.

Die Gelpermeationschromatographie (GPC) ist eine Relativmethode bei der die Moleküle aufgrund ihrer Größe, genauer gesagt aufgrund ihres hydrodynamischen Volumens, getrennt werden. Die Bestimmung der absoluten Molmasse erfolgt durch Kalibrierung mit bekannten Standards. Als Kalibrierstandard werden vorzugsweise engverteile Polystyrolstandards eingesetzt. Diese sind kommerziell erhältlich. Als Trennmaterial werden Styrol-Divinylbenzol-Säulen und Tetrahydrofuran (THF) als Elutionsmittel verwendet. Styrol-Divinylbenzol-Säulen eignen sich für organische, lösliche, synthetische Polymere. Die Messung erfolgt mit verdünnten Lösungen (0,05 bis 0,2 Gew.-%) der zu untersuchenden Polymere.

Alternativ kann die zahlenmittlere Molmasse mit den bekannten Methoden der Gefrierpunktserniedrigung (Kryoskopie), der Siedepunktserhöhung (Ebullioskopie) oder aus der Erniedrigung des Dampfdrucks (Dampfdruckosmometrie) bestimmt werden. Hierbei handelt es sich um absolute Methoden, die keine Kalibrierstandards erfordern. Es werden Konzentrationsreihen von 4 bis 6 verdünnten Polymerlösungen mit Konzentrationen von 0,005 bis 0,10 mol/kg untersucht und dann die gemessenen Werte auf eine Konzentration von 0 mol/kg extrapoliert.

Erfindungsgemäß sind Zusammensetzungen bevorzugt, die
(a) 1 bis 30 Gew.-%, bevorzugt 2 bis 25 Gew.-% und besonders bevorzugt 3 bis 20 Gew.-% mindestens eines Übertragungsreagenzes der Formel I,
(b) 1 bis 97,9 Gew.-% mindestens eines urethangruppenhaltigen (Meth)acrylatmonomers, vorzugsweise Urethandi(meth)acrylats,
(c) 0 bis 20 Gew.-% eines oder mehrerer Mono(meth)acrylate,
(d) 0 bis 50 Gew.-% eines oder mehrerer Di(meth)acrylate ohne Urethangruppen,
(e) 1 bis 10 Gew.-% mindestens eines Bruchzähigkeitsmodifikators,
(f) ggf. 10 bis 5000 ppm eines oder mehrerer Inhibitoren,
(g) 0,1 bis 5,0 Gew.-% mindestens eines Photoinitiators für die radikalische Polymerisation,
(h) 0 bis 30 Gew.-% eines oder mehrerer anorganischer oder organischer Füllstoffe,
(i) 0 bis 30 Gew.-% eines oder mehrerer Additive enthalten.

Alle Prozentangaben beziehen sich, wenn nicht anders angegeben, auf die Gesamtmasse der Zusammensetzung.

Besonders bevorzugt sind Zusammensetzungen, die
(a) 1 bis 30 Gew.-%, bevorzugt 2 bis 25 Gew.-% und besonders bevorzugt 3 bis 20 Gew.-% mindestens eines Übertragungsreagenzes der Formel I,
(b) 1 bis 97,9 Gew.-%, bevorzugt 20 bis 95,84 Gew.-% und besonders bevorzugt 30 bis 93,78 Gew.-% mindestens eines Urethandi(meth)acrylats,
(c) 0 bis 20 Gew.-%, bevorzugt 0 bis 10 Gew.-% und besonders bevorzugt 0 Gew.- % eines oder mehrerer Mono(meth)acrylate,
(d) 0 bis 50 Gew.-%, bevorzugt 0 bis 40 Gew.-% und besonders bevorzugt 0 bis 30 Gew.-% eines oder mehrerer Di(meth)acrylate ohne Urethangruppen,
(e) 1 bis 10 Gew.-%, bevorzugt 2 bis 8 Gew.-% und besonders bevorzugt 3 bis 6 Gew.-% mindestens eines Bruchzähigkeitsmodifikators,
(f) ggf. 10 bis 5000 ppm, bevorzugt 100 bis 4000 ppm und besonders bevorzugt 150 bis 1500 ppm eines oder mehrerer phenolischer Inhibitoren,
(g) 0,1 bis 5,0 Gew.-%, bevorzugt 0,15 bis 4,0 Gew.-% und besonders bevorzugt 0,2 bis 3,0 Gew.-% mindestens eines Photoinitiators für die radikalische Polymerisation,
(h) 0 bis 30 Gew.-%, bevorzugt 0 bis 20 Gew.-% und besonders bevorzugt 0 bis 10 Gew.-% eines oder mehrerer anorganischer oder organischer Füllstoffe,
(i) 0 bis 30 Gew.-%, bevorzugt 0 bis 25 Gew.-% und besonders bevorzugt 0 bis 20 Gew.-% an weiteren Additiven enthalten.

Die erfindungsgemäßen Zusammensetzungen können bis zu 20 Gew.-% an **Mono(meth)acrylaten (c)** enthalten, wobei das oder die Mono(meth)acrylate vorzugsweise aus aliphatischen, cycloaliphatischen, aromatischen, bicyclischen und tricyclischen Mono(meth)acrylate ausgewählt sind und die Gesamtmenge an Mono(meth)acrylaten die genannten Mengenbereiche nicht überschreitet. Besonders bevorzugte sind aliphatische Mono(meth)acrylate oder Mono(meth)acrylate mit einer cycloaliphatischen, aromatischen, bicyclischen oder tricyclischen Gruppe, wie z.B. Benzyl-, Tetrahydrofurfuryl- oder Isobornyl(meth)acrylat, 2-[(Methoxycarbonyl)amino]ethylmethacrylat, 2-[(Propoxycarbonyl)amino]ethylmethacrylat, 2-[(Isopropoxycarbonyl)amino]-ethylmethacrylat, 2-[(Butoxycarbonyl)amino]ethylmethacrylat, 2-[(Hexyloxycarbonyl)-amino]-ethylmethacrylat, 2-[(Cyclohexyloxycarbonyl)amino]-ethylmethacrylat, 2-[(Ethylcarbamoyl)oxy]ethylmethacrylat, 2-[(Propylcarbamoyl)oxy]ethylmethacrylat, 2-[(lsopropylcarbamoyl)oxy]ethylmethacrylat, 2-[(Hexylcarbamoyl)oxy]ethylmethacrylat, 2-[(2-Tetrahydrofurfuryloxycarbonyl)amino]ethylmethacrylat, 2-(2-Oxo-[1,3]-dioxolan-4-ylmethoxycarbonylamino)ethylmethacrylat, Methacrylsäure-2-(furan-2-yl-methoxy-carbonylamino)ethylester, (1,3-Dioxolanon-2-on-4-yl)methylmethacrylat, 2-Phenoxyethyl(meth)acrylat, 2-(o-Biphenyloxy)ethyl(meth)acrylat, 2-Hydroxy-3-phenoxypropyl-(meth)acrylat, 2-[(Benzyloxycarbonyl)amino]ethyl(meth)acrylat, 2-[(Benzylcarbamoyl)-oxy]ethyl(meth)acrylat, 1-Phenoxypropan-2-yl-(meth)acrylat und 2-(p-Cumylphenoxy)-ethyl(meth)acrylat, Tricylodecan-(meth)acrylat, Tricyclodecanmethyl(meth)acrylat , 4,7,7-Trimethylbicyclo[2.2.1]heptanyl(meth)acrylat und Mischungen davon.

Erfindungsgemäß sind schwerflüchtige Mono(meth)acrylate (c) bevorzugt. Unter schwerflüchtigen Stoffen werden Verbindungen mit einer Verdunstungszahl von größer 35 verstanden. Die Verdunstungszahl (VD) gibt an, wie schnell ein Stoff bei Raumtemperatur verdunstet. Sie wird nach der DIN 53170 bestimmt. Dabei wird die Zeit, in der ein Stoff komplett verdunstet (Verdunstungszeit = VDZ) mit der Zeit in Relation gesetzt, die Diethylether zum Verdunsten benötigt. Je höher die Verdunstungszahl ist, desto langsamer verdunstet ein Stoff.

Ganz besonders bevorzugte monofunktionelle Monomere sind Tetrahydrofurfuryl- und Isobornyl(meth)acrylat, 2-[(Benzyloxycarbonyl)amino]ethylmethacrylat, 2-[(Ethyl-carbamoyl)oxy]-ethylmethacrylat, 2-[(Benzylcarbamoyl)-oxy]ethylmethacrylat, Methacrylsäure-2-(furan-2-yl-methoxycarbonylamino)-ethylester, 2-Phenoxyethyl(meth)acrylat, 2-(o-Biphenyloxy)ethyl(meth)acrylat, 2-Hydroxy-3-phenoxypropyl(meth)acrylat, 1-Phenoxypropan-2-yl-(meth)acrylat, 2-(p-Cumylphenoxy)ethyl(meth)acrylat, Tricylo-decan(meth)acrylat, Tricyclodecanmethyl(meth)acrylat, 4,7,7-Trimethylbicyclo[2.2.1]-heptanyl(meth)acrylat und Mischungen davon.

Aliphatische, cycloaliphatische, aromatische, bicyclische und tricyclische Mono(meth)acrylate (c) zeichnen sich durch eine gute radikalische Polymerisationsfähigkeit aus. Darüber hinaus haben die Polymerisate dieser Mono(meth)acrylate einen vergleichsweise geringen Polymerisationsschrumpf und gute mechanische Eigenschaften. Aufgrund ihrer relativ hohen Molmasse (150 bis 350 g/mol) und ihrer relativ unpolaren Struktur haben die erfindungsgemäß bevorzugten Mono(meth)acrylate außerdem eine geringe Flüchtigkeit und eine vergleichsweise geringe Viskosität. Allerdings führen Mono(meth)acrylate zu einer Verringerung der Netzwerkdichte. Der Anteil an Mono(meth)acrylaten ist daher auf maximal 20 Gew.-% und vorzugsweise maximal 10 Gew.-% beschränkt. Besonders bevorzugt sind Zusammensetzungen, die keine monofunktionellen Monomere enthalten.

Die erfindungsgemäßen Zusammensetzungen können als **Komponente (d)** 0 bis 50 Gew.-% eines oder mehrerer Di(meth)acrylate ohne Urethangruppen enthalten. Bevorzugte Di(meth)acrylate (d) sind Bisphenol-A-di(meth)acrylat, Bis-GMA (ein Additionsprodukt aus (Meth)acrylsäure und Bisphenol-A-diglycidylether), ethoxy- oder propoxyliertes Bisphenol-A-di(meth)acrylat oder 2,2-Bis[4-(2-(meth)acryloyloxypropoxy)-phenyl]propan, Tri- oder Tetraethylenglycoldi(meth)acrylat, 1,10-Decandioldi(meth)acrylat, Bis[(meth)acryloyloxymethyl]tricyclo-[5.2.1.0^{2,6}]decan oder 1,12-Dodecandioldi-(meth)acrylat.

Die Di(meth)acrylate (d) ermöglichen eine weitere Einstellung der Vernetzungsdichte und eine Beeinflussung der mechanischen Eigenschaften der Polymerisate. Besonders bevorzugte Di(meth)acrylate (d) sind Bis-GMA, Bisphenol-A-di(meth)acrylat mit 3 Ethoxygruppen, 2,2-Bis[4-(2-(meth)acryloyloxypropoxy)phenyl]propan, Triethylengly-coldi(meth)acrylat, sowie Glycerintri(meth)acrylat, 1,10-Decandioldi(meth)acrylat, Bis-[(meth)acryloyloxymethyl]tricyclo-[5.2.1.0^{2,6}]decan, 1,12-Dodecandioldi(meth)acrylat und Mischungen davon.

Die bevorzugten Di(meth)acrylatmonomere (d) zeichnen sich durch ein relativ geringes Mol-Gewicht aus, wobei Di(meth)acrylate (d) mit einem Mol-Gewicht im Bereich von 200 bis 800 g/mol und insbesondere 220 bis 650 g/mol besonders bevorzugt sind.

Wegen des, insbesondere im Vergleich zu den Urethandi(meth)acrylat-Telechelen (b), geringen Mol-Gewichts bewirken die Di(meth)acrylatmonomere (d) eine relativ starke Vernetzung der Polymerisate. Der Anteil weiterer Di(meth)acrylate (d) ist daher auf maximal 50 Gew.-%, vorzugsweise maximal 30 Gew.-% beschränkt. Gemäß einer besonders bevorzugten Ausführungsform enthalten die erfindungsgemäßen Dentalwerkstoffe als Vernetzer ausschließlich urethangruppenhaltige (Meth)acrylatmonomere (b), vorzugsweise Urethandi(meth)acrylate. Weiterhin ist es bevorzugt, dass die erfindungsgemäßen Zusammensetzungen neben den urethangruppenhaltigen (Meth)acrylatmonomeren (b) und den Di(meth)acrylaten (d) keine weiteren polyfunktionellen Monomere enthalten, wie beispielsweise Tri-, Tetra- oder höherfunktionelle (Meth)acrylate.

Urethangruppenhaltige (Meth)acrylatmonomere (b), monofunktionelle Methacrylate (c), und Di(meth)acrylatmonomere (d) werden vorzugsweise in einem solchen Mengenverhältnis eingesetzt, dass Polymere mit einer Netzwerkdichte von vc = 800 bis 16.000 mol/m³, bevorzugt 2000 bis 10.000 mol/m³ besonders bevorzugt 2500 bis 8000 mol/m³ erhalten werden. Die Vernetzungsdichte wird erfindungsgemäß maßgeblich durch den Gehalt an Übertragungsreagenz (a) eingestellt. Daneben wird die Vernetzungsdichte aber auch durch das Verhältnis von vernetzenden zu monofunktionellen Monomeren beeinflusst. Erfindungsgemäß bevorzugt sind Dentalwerkstoffe, in denen der Molenbruch der vernetzenden Monomeren in einem Bereich von 0,80 bis 1.0 liegt. Besonders bevorzugt sind Harze, die keine monofunktionellen Monomere enthalten.

Zur Berechnung des Molenbruchs werden alle radikalisch polymerisierbaren Komponenten der erfindungsgemäßen Werkstoffe herangezogen, d.h. insbesondere die Komponenten (b), (c) und (d). Unter vernetzenden Monomeren werden alle radikalisch polymerisierbaren Komponenten verstanden, die zwei oder mehr radikalisch polymerisierbare Gruppen aufweisen, d.h. insbesondere die Komponenten (b) und (d). Vernetzende Monomere werden auch als polyfunktionelle Monomere bezeichnet. Monofunktionelle Monomere sind Monomere mit nur einer radikalisch polymerisierbaren Gruppe.

Die Netzwerkdichte entspricht der Zahl der Netzknoten (in mol) je Volumeneinheit und lässt sich durch dynamisch-mechanische Messungen aus dem Plateauwert des Speichermoduls G' im elastischen Bereich berechnen. Die Glasübergangstemperatur T_{g} und die Netzwerkdichte v_{c} werden mit einem Rheometer bestimmt, vorzugsweise einem Anton Paar Rheometer MCR301. Hierzu werden Speicher- und Verlustmodul eines Prüfkörpers (25 x 5 x 1 mm, längs eingespannt) zwischen 25°C und 250°C vermessen (Frequenz 1 Hz, Deformation 0,05 %, Heizrate 2K / min). T_{g} ergibt sich dabei als das Maximum des Verlustfaktors tan δ (Verhältnis von Verlustmodul zu Speichermodul). Die Netzwerkdichte wird nach der Formel v_{c} = G'/(RT) berechnet, mit G' als dem Speichermodul bei der Temperatur T_{g} + 50 K, R als der allgemeinen Gaskonstante und T als der Temperatur bei T_{g} + 50 K in Kelvin.

Die erfindungsgemäßen Zusammensetzungen enthalten vorzugsweise mindestens einen **Inhibitor (f),** besonders bevorzugt in einer Menge von 10 bis 5000 ppm, ganz besonders bevorzugt in einer Menge von 100 bis 4000 ppm und am meisten bevorzugte von 150 bis 1500 ppm, bezogen auf die Gesamtmasse der Zusammensetzung. Unter Inhibitoren werden radikalfangende Stoffe zur Verhinderung einer vorzeitigen Polyreaktion verstanden. Die Inhibitoren werden auch als Polymerisationsinhibitoren bezeichnet. Sie verbessern die Lagerstabilität der Materialien. Bevorzugte Inhibitoren sind phenolische Inhibitoren, insbesondere Hydrochinonmonomethylether (MEHQ, CAS: 150-76-5), 2,6-Di-tert.-butyl-4-methyl-phenol (BHT, CAS: 128-37-0), 2,6-Di-tert.-butylphenol (CAS: 128-39-2), Pyrogallol (CAS: 87-66-1), Hydrochinon (CAS: 123-31-9), 2-tert.-Butylhydrochinon (CAS: 1948-33-0), 4-tert.-Butylbrenzcatechin (CAS: 98-29-3) und 6-tert.-Butyl-2,4-xylenol (CAS: 1879-09-0), wobei MEHQ, Pyrogallol und BHT besonders bevorzugt sind.

Es wurde überraschend gefunden, dass die bevorzugten phenolischen Inhibitoren, insbesondere in einer Menge von 150 bis 1500 ppm, die erfindungsgemäßen Zusammensetzungen einerseits sehr gut vor einer vorzeitigen Polymerisation schützen und andererseits eine gute Polymerisationsreaktivität gewährleisten. Sie führen nach der Härtung zu Materialien mit hoher Biegefestigkeit, hohem Biegemodul und großer Brucharbeit.

Die erfindungsgemäßen Zusammensetzungen enthalten mindestens einen **Photoinitiator (g).** Es können Photoinitiatoren für den UV- oder vorzugsweise den sichtbaren Wellenlängenbereich des Lichts eingesetzt werden.

Bevorzugte Photoinitiatoren für den sichtbaren Bereich sind α-Diketone und deren Derivate, wie 9,10-Phenanthrenchinon, 1-Phenyl-propan-1,2-dion, Diacetyl oder 4,4'-Dichlorbenzil. Besonders bevorzugt werden Campherchinon (CQ) und 2,2-Dimethoxy-2-phenyl-acetophenon und ganz besonders bevorzugt α-Diketone in Kombination mit Aminen als Reduktionsmittel, wie z.B. 4-(Dimethylamino)-benzoesäureester (ED-MAB), N,N-Dimethylaminoethylmethacrylat, N,N-Dimethyl-3,5-xylidin oder Triethanolamin, verwendet. Weitere bevorzugte Photoinitiatoren für den sichtbaren Bereich sind monomolekulare Photoinitiatoren, sog. Norrish-Typ-I-Photoinitiatoren, insbesondere Monoacyltrialkyl-, Diacyldialkyl- und Tetraacylgermanium sowie Tetraacylstannane, wie z.B. Benzoyltrimethylgermanium, Dibenzoyldiethylgermanium, Bis(4-methoxybenzoyl)diethylgermanium, Tetrakis(2-methylbenzoyl)germanium oder Tetrakis-(mesitoyl)stannan. Dabei lassen sich auch Mischungen der verschiedenen Photoinitiatoren einsetzen, wie z.B. Bis(4-methoxybenzoyl)diethylgermanium in Kombination mit Campherchinon und 4-Dimethylaminobenzoesäureethylester.

Besonders bevorzugte Photoinitiatoren für den UV- und sichtbaren Bereich sind Monoacylphosphine wie Diphenyl-(2,4,6-trimethylbenzoyl)phosphinoxid (Lucirin^{®} TPO, CAS: 75980-60-8) oder 2,4,6-Trimethylbenzoylethoxylphenylphosphinoxid (Lucirin^{®} TPO-L CAS: 84434-11-7) oder andere 2,4,6-Trimethylbenzoylalkoxyphenylphosphinoxide und weiter Bisacylphosphinoxide, wie z.B. Irgacure 819 (Bis(2,4,6-trimethylbenzoyl)phenylphosphinoxid) (CAS: 16881-26-7). Ganz besonders bevorzugte Photoinitiatoren sind Diphenyl-(2,4,6-trimethylbenzoyl)phosphinoxid und 2,4,6-Trimethylbenzoylethoxylphenylphosphinoxid.

Bevorzugte UV-Photoinitiatoren sind Acetophenone, z.B. 2,2-Diethoxy-1-phenylethanon, Benzoinether, wie z.B. Irgacure 651 (Dimethylbenzilketal), Hydroxyalkylphenylacetophenone, wie z.B. Irgacure 184 (1-Hydroxy-cyclohexyl-phenyl-keton), 2-Benzyl-2-(dimethylamino)-4'-morpholinobutyrophenon (Irgacure 369) und 1-Butanon-2-(dimethylamino)-2-(4-methylphenyl)methyl-1-4-(4-morpholinyl)phenyl (Irgacure 379).

Für eine Nachvergütung ist es von Vorteil, zwei Photoinitiatoren einzusetzen, die sich durch ihre Absorptionsbereiche unterscheiden. Bevorzugt sind Mischungen von Photoinitiatoren für den UV-Bereich und den sichtbaren Bereich. Unter einer Nachvergütung von radikalischen Polymerisaten versteht man eine zusätzliche Bestrahlung der Polymerisate und/oder nachträgliche Wärmebehandlung zur Erhöhung des Doppelbindungs- bzw. Monomerumsatzes und damit zur Verbesserung der mechanischen und optischen Eigenschaften der Polymerisate.

Die erfindungsgemäßen Dentalwerkstoffe können zusätzlich auch einen oder mehrere thermische Initiatoren enthalten, z.B. Azoverbindungen, wie 2,2'-Azobis(isobutyronitril) (AIBN) oder Azobis-(4-cyanovaleriansäure), oder Peroxide, wie Dibenzoylperoxid, Dilauroylperoxid, *tert*-Butylperoctoat, *tert*-Butylperbenzoat oder Di-(*tert*-butyl)-peroxid. Zur Beschleunigung der Initiierung mittels Peroxiden lassen sich auch Kombinationen mit aromatischen Aminen einsetzen. Bevorzugte Redoxsysteme sind Kombinationen von Dibenzoylperoxid mit Aminen, wie N,N-Dimethyl-p-toluidin, N,N-Dihydroxyethyl-p-toluidin, p-Dimethylaminobenzoesäureethylester oder strukturverwandte Systeme.

Die erfindungsgemäßen Zusammensetzungen können außerdem einen oder mehrere **Füllstoffe (H)** enthalten, vorzugsweise partikuläre oder faserförmige anorganische oder organische Füllstoffe oder Kompositfüllstoffe. Füllstoffe ermöglichen eine Beeinflussung der mechanischen Eigenschaften. Besonders bevorzugt sind anorganische partikuläre Füllstoffe.

Bevorzugte anorganische Füllstoffe sind Oxide, wie SiO₂, ZrO₂ und TiO₂ oder Mischoxide aus SiO₂, ZrO₂, ZnO und/oder TiO₂, nanopartikuläre oder mikrofeine Füllstoffe, wie pyrogene Kieselsäure oder Fällungskieselsäure, Glaspulver, wie Quarz-, Glaskeramik-, Borosilikat- oder röntgenopake Glaspulver, vorzugsweise Barium- oder Strontiumaluminiumsilikatgläser, und röntgenopake Füllstoffe, wie Ytterbiumtrifluorid, Tantal(V)-oxid, Bariumsulfat oder Mischoxide von SiO₂ mit Ytterbium(III)-oxid oder Tantal(V)-oxid. Weiterhin können die erfindungsgemäßen Dentalwerkstoffe faserförmige Füllstoffe, Nanofasern, Whiskers oder Mischungen davon enthalten. Gemäß einer bevorzugten Ausführungsform enthalten die erfindungsgemäßen Werkstoffe keine Fluoroaluminosilikatgläser, Calciumaluminiumsilikatgläser oder andere Füllstoffe, die mit organischen Säuren im Sinne einer Säure-Base-Reaktion reagieren.

Bevorzugt weisen die Oxide eine Partikelgröße von 0,010 bis 15 µm , die nanopartikulären oder mikrofeinen Füllstoffe eine Partikelgröße von 10 bis 300 nm, die Glaspulver eine Partikelgröße von 0,10 bis 15 µm, vorzugweise von 0,2 bis 1,5 µm, und die röntgenopaken Füllstoffe eine Partikelgröße von 0,2 bis 5 µm auf.

Besonders bevorzugte Füllstoffe sind Mischoxide aus SiO₂ und ZrO₂, mit einer Partikelgröße von 10 bis 300 nm, Glaspulver mit einer Partikelgröße von 0,2 bis 1,5 µm, insbesondere röntgenopake Glaspulver z.B. von Barium- oder Strontiumaluminiumsilikatgläsern, und röntgenopake Füllstoffe mit einer Partikelgröße von 0,2 bis 5 µm , insbesondere Ytterbiumtrifluorid und/oder Mischoxide von SiO₂ mit Ytterbium(III)- oxid.

Zur Verbesserung des Verbundes zwischen den Füllstoffpartikeln und der vernetzten Polymerisationsmatrix können SiO₂-basierende Füllstoffe mit methacrylatfunktionalisierten Silanen oberflächenmodifiziert werden. Ein bevorzugtes Beispiel für solche Silane ist 3-Methacryloyloxypropyltrimethoxysilan. Zur Oberflächenmodifizierung von nichtsilikatischen Füllstoffen wie ZrO₂ oder TiO₂ können auch funktionalisierte saure Phosphate, wie z.B. 10-Methacryloyloxydecyldihydrogenphosphat eingesetzt werden.

Weitere bevorzugte Füllstoffe sind partikulare Wachse, insbesondere Carnaubawachs, vorzugsweise mit eine Partikelgröße von 1 bis 10 µm, unvernetzte oder teilvernetzte Polymethylmethacrylat (PMMA)-Partikel, vorzugsweise mit einer Partikelgröße von 500 nm bis 10 µm, sowie Polyamid-12 Partikel, vorzugsweise mit einer Partikelgröße 5 bis 10 µm.

Außerdem können die erfindungsgemäßen Dentalwerkstoffe sogenannte Kompositfüllstoffe oder Isofüllstoffe enthalten, d.h. gemahlene Komposite, die vorzugsweise eine breite Partikelgrößenverteilung z.B. mit Partikelgrößen von 0,05 bis 20 µm, insbesondere etwa 0,1 bis etwa 10 µm, aufweisen. Vorzugsweise ist der Kompositfüllstoff oder Isofüllstoff oberflächenmodifiziert, insbesondere silanisiert.

Wenn nicht anders angegeben, handelt es sich bei allen Partikelgrößen hierin um gewichtsmittlere Partikelgrößen, wobei die Partikelgrößenbestimmung im Bereich von 0,1 µm bis 1000 µm mittels statischer Lichtstreuung erfolgt, vorzugsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5 %ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320.

Partikelgrößen kleiner als 0,1 µm werden vorzugsweise mittels Dynamischer Lichtstreuung (DLS) bestimmt. Die Messung der Partikelgröße im Bereich von 5 nm bis 0,1 µm erfolgt vorzugsweise durch Dynamische Lichtstreuung (DLS) von wässrigen Partikeldispersionen, vorzugsweise mit einem Malvern Zetasizer Nano ZS (Malvern Instruments, Malvern UK) mit einem He-Ne-Laser mit einer Wellenlänge von 633 nm, bei einem Streuwinkel von 90° bei 25°C

Die Lichtstreuung nimmt mit abnehmender Partikelgröße ab. Partikelgrößen kleiner als 0,1 µm können auch mittels REM- oder TEM-Spektroskopie bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf einem 50 A dickem Kupfergitter (Maschenweite: 300 Mesh) aufgebracht, das mit Kohlenstoff beschichtet ist, und im Anschluss das Lösungsmittel verdampft.

Die Füllstoffe werden nach ihrer Partikelgröße unterteilt in Makrofüller und Mikrofüller, wobei Füllstoffe mit einer mittleren Partikelgröße von 0,2 bis 10 µm als Makrofüller und Füllstoffe mit einer mittlere Partikelgröße von ca. 5 bis 100 nm als Mikrofüller bezeichnet werden. Makrofüller werden z.B. durch Mahlen z.B. von Quarz, röntgenopaken Gläsern, Borosilikaten oder von Keramik gewonnen und bestehen meist aus splitterförmigen Teilen. Als Mikrofüller werden vorzugsweise pyrogenes SiO₂ oder Fällungskieselsäure eingesetzt, oder Mischoxide, z.B. SiO₂-ZrO₂, die durch hydrolytische Cokondensation von Metallalkoxiden zugänglich sind. Die Mikrofüller haben vorzugsweise eine mittlere Partikelgröße von ca. 5 bis 100 nm. Mikrofüller. Füllstoffe mit geringer Partikelgröße haben eine größere Verdickungswirkung.

In einer bevorzugten Ausführungsform enthalten die erfindungsgemäßen Zusammensetzungen eine Mischung von zwei oder mehreren Füllstoffen, insbesondere von zwei oder mehreren Füllstoffen mit unterschiedlichen Partikelgrößen. Es wurde gefunden, dass durch die Verwendung solcher Füllstoffmischungen die Viskosität der Werkstoffe nicht übermäßig erhöht wird und die Zusammensetzungen daher mit generativen Verfahren, wie z.B. mit Hilfe der Stereolithographie, gut verarbeitbar sind. Der Gesamtgehalt an Füllstoffen liegt vorzugsweise in einem Bereich von 0 bis 30 Gew.-%, besonders bevorzugt von 0 bis 10 Gew.-%.

Die erfindungsgemäßen Zusammensetzungen können darüber hinaus ein oder mehrere **Additive (i)** enthalten, insbesondere UV-Absorber, optische Aufheller, Farbmittel, Weichmacher und/oder Thixotropieadditive.

Die Zusammensetzungen können einen oder mehrere **UV-Absorber** enthalten. Der UV-Absorber dient dazu, bei der lichtinduzierten Härtung der erfindungsgemäßen Zusammensetzung die Eindringtiefe des Lichts und damit die Polymerisationstiefe zu verringern. Dies erweist sich insbesondere bei stereolithographischen Anwendungen als vorteilhaft, da bei der Stereolithographie lediglich dünne Schichten gehärtet werden sollen. Durch Verwendung eines UV-Absorbers kann bei stereolithographischen Verfahren die Präzision verbessert werden.

Bevorzugt sind UV-Absorber auf der Basis von Benzotriazol, Benzophenon oder Triazine. Besonders bevorzugte UV-Absorber sind 2,2'-Methylenbis[6-(2H-benzotriazol-2-yl)-4-(1,1,3,3-tetramethylbutyl)phenol], 2,2',4,4'-Tetrahydroxybenzophenon, 2-tert-Butyl-6-(5-chloro-2H-benzotriazol-2-yl)-4-methylphenol (Bumetrizol), 2,2'-Benzol-1,4-diylbis(4h-3,1-benzoxazin-4-on), 2-(4,6-Bis-(2,4-dimethylphenyl)-1,3,5-triazin-2-yl)-5-(octyloxy)-phenol, 2-(2-Hydroxy-5-methylphenyl)benzotriazol, 2-(2-Hydroxyphenyl)-benzotriazol, 2-(2H-Benzotriazol-2-yl)-6-dodecyl-4-methylphenol, 2-(2'-Hydroxy-3', 5'-di-t-butylphenyl)-5-chlorobenzotriazole, 2,2'-Dihydroxy-4-methoxybenzophenon und 2,2'-Dihydroxy-4,4'-dimethoxybenzophenon. Weiter bevorzugt sind sogenannte Hindered Amine Light Stabilizers wie Bis(1,2,2,6,6-pentamethyl-4-piperidyl)sebacat, Methyl-1,2,2,6,6-pentamethyl-4-piperidylsebacat, Bis(1-octyloxy-2,2,6,6-tetramethyl-4-piperidyl)sebacat und Bis(1,2, 2,6,6-pentamethyl-4-piperidyl)-[[3,5-bis(1,1-dimethylethyl)-4-hydroxyphenyl]methyl]butylmalonat. Ganz besonders bevorzugte UV-Absorber sind Bumetrizol und 2,2',4,4'-Tetrahydroxybenzophenon.

Der UV-Absorber weist vorzugsweise ein Absorptionsmaximum auf, das der Wellenlänge des zur Härtung eingesetzten Lichts entspricht. Vorteilhaft sind UV-Absorber mit einem Absorptionsmaximum im Bereich von 320 bis 500 nm und vorzugsweise 380 bis 480 nm, wobei UV-Absorber mit einem Absorptionsmaximum unterhalb von 400 nm besonders bevorzugt sind.

UV-Absorber werden ggf. in einer Menge von vorzugsweise bis zu 0,5 Gew.-% eingesetzt. Bumetrizol wird vorzugsweise in einer Menge von bis zu 0,15 Gew.-%, und 2,2',4,4'-Tetrahydroxybenzophenon in einer Menge bis zu 0,07 Gew.-% verwendet. Alle Angaben beziehen sich auf das Gesamtgewicht des Werkstoffs. Dentalwerkstoffe, die keinen UV-Absorber enthalten, sind besonders bevorzugt.

Die erfindungsgemäßen Zusammensetzungen können einen oder mehrere **optische Aufheller** enthalten. Erfindungsgemäß bevorzugt sind optische Aufheller, die Licht im UV-Bereich absorbieren, d.h. Licht mit einer Wellenlänge unterhalb von 400 nm. Durch die Zugabe eines optischen Aufhellers kann die Eindringtiefe des Lichts und damit die Durchhärtungstiefe verringert und so die Präzision bei stereolithographischen Prozessen erhöht werden. Besonders bevorzugt sind optische Aufheller, die in der Lage sind, das im UV-Bereich absorbierte Licht als Licht mit einer Wellenlänge von 400 bis 450 nm wieder zu emittieren. Solche optischen Aufheller erhöhen die Reaktivität der Werkstoffe, indem sie das aufgenommene kurzwellige Licht aufgrund ihrer Fluoreszenz als längerwelliges Blaulicht abstrahlen und damit zusätzliche Lichtleistung zur Photoinitiierung bereitstellen. Erfindungsgemäß bevorzugte optische Aufheller sind 2,5-Bis(5-tert-butyl-benzoxazol-2-yl)thiophen und Fluoreszenzmittel in Form von Terephthalsäurederivaten, wie z.B. 2,5-Dihydroxyterephthalsäurediethylester oder Diethyl-2,5-dihyd-roxyterephthalat.

Der oder die optischen Aufheller werden ggf. in einer Menge von vorzugsweise bis zu 0,05 Gew.-% jeweils bezogen auf das Gesamtgewicht des Dentalwerkstoffe eingesetzt. Zusammensetzungen, die keinen optischen Aufheller enthalten, sind besonders bevorzugt.

Optische Aufheller können in Kombination mit UV-Absorbern eingesetzt werden. In diesem Fall ist es bevorzugt, dass das Gewichtsverhältnis von UV-Absorber zu optischem Aufheller in einem Bereich von 2:1 bis 50:1, besonders bevorzugt 2:1 bis 30:1 und ganz besonders bevorzugt 2:1 bis 5:1 oder 10:1 bis 25:1 liegt. Bevorzugt sind Kombinationen, die als UV-Absorber 2,2',4,4'-Tetrahydroxybenzophenon oder Bumetrizol und als optischen Aufheller 2,5-Bis(5-tert-butyl-benzoxazol-2-yl)thiophen enthalten. Ganz besonders bevorzugt ist die Kombination von 2,2',4,4'-Tetrahydroxybenzophenon und 2,5-Bis(5-tert-butyl-benzoxazol-2-yl)thiophen in einem Gewichtsverhältnis von 2:1 bis 10:1, vorzugsweise 2:1 bis 5:1, oder die Kombination von Bumetrizol und 2,5-Bis(5-tert-butyl-benzoxazol-2-yl)thiophen in einem Gewichtsverhältnis von 5:1 bis 30:1, vorzugsweise 10:1 bis 20:1.

Die erfindungsgemäßen Zusammensetzungen können ein oder mehrere **Farbmittel** enthalten, vorzugsweise in einer Konzentration bis von 0 bis 0,5 Gew.-%. Die Farbmittel dienen primär ästhetischen Zwecken. Erfindungsgemäß bevorzugte Farbmittel sind organische Farbstoffe und Pigmente, insbesondere Azofarbstoffe, Carbonylfarbstoffe, Cyaninfarbstoffe, Azomethine und Methine, Phthalocyanine und Dioxazine. Besonders bevorzugt sind Farbstoffe, die in den erfindungsgemäßen Werkstoffen löslich sind, insbesondere Azofarbstoffe. Als Farbmittel eignen sich Außerdem anorganische und insbesondere organische Pigmente, die sich gut in den erfindungsgemäßen Dentalwerkstoffe dispergieren lassen. Bevorzugte anorganische Pigmente sind Metalloxide oder Hydroxide, wie z.B. Titandioxid oder ZnO als Weisspigmente, Eisenoxid (Fe₂O₃) als Rotpigment oder Eisenhydroxid (FeOOH) als Gelbpigment. Bevorzugte organische Pigmente sind Azopigmente, wie z.B. Monoazogelb und Orangepigmente, Diazopigmente oder β-Naphthol-Pigmente, und Nichtazo- oder polycyclische Pigmente, wie z.B. Phthalocyanin-, Chinacridon-, Perylen- und Flavanthron-Pigmente. Besonders bevorzugt sind Azopigmente und Nichtazopigmente.

Außerdem können erfindungsgemäßen Zusammensetzungen einen oder mehrere **Weichmacher** enthalten. Weichmacher verhindern, dass die Polymerisate nach der photochemischen Aushärtung und einem evtl. Trocknen brüchig werden. Darüber hinaus sorgen Weichmacher für eine ausreichende Flexibilität. Weichmacher werden vorzugsweise in einer Konzentration von 0,2 bis 5 Gew.-% zugesetzt. Bevorzugte Weichmacher sind Phthalate, wie z.B. Dibutyl- oder Dihexylphthalat, nichtsaure Phosphate, wie z.B. Tributyl- oder Trikresylphosphat, n-Octanol, Glycerin oder Polyethylenglykole. Besonders bevorzugt sind Weinsäure- oder Citronsäureester, wie z.B. Citronensäuretriester, die sich durch eine gute Biokompatibilität auszeichnen.

Die erfindungsgemäßen Zusammensetzungen können zudem ein oder mehrere Thixotropieadditive enthalten. Diese Additive bewirken eine Verdickung der Werkstoffe und können so beispielsweise ein Sedimentieren der Füllstoffe verhindern. Insbesondere füllstoffhaltige Zusammensetzungen enthalten daher vorzugweise mindestens ein Thixotropieadditiv. Bevorzugte Thixotropieadditive sind OH-Gruppen-haltige Polymere, wie z.B. Cellulosederivate, und anorganische Stoffe, wie z.B. Schichtsilicate. Um die Viskosität der Werkstoffe nicht zu stark zu erhöhen, enthalten die erfindungsgemäßen Dentalwerkstoffe vorzugsweise nur 0 bis 3,0 Gew.-%, besonders bevorzugt 0 bis 2,0 Gew.-% und ganz besonders bevorzugt 0,1 bis 2,0 Gew.-% Thixotropieadditiv, bezogen auf das Gesamtgewicht der Zusammensetzung.

Bestimmte Füllstoffe, wie z.B. hochdisperses SiO₂, d.h. SiO₂ mit geringer Primärteilchengröße (< 20 nm) und hoher spezifischer Oberfläche (> 100 m²/g) haben ebenfalls eine thixotropierende Wirkung. Solche Füllstoffe können Thixotropieadditive ersetzen.

Die rheologischen Eigenschaften der erfindungsgemäßen Zusammensetzungen werden an den gewünschten Anwendungszweck angepasst. Materialen zur stereolithographischen Verarbeitung werden vorzugsweise so eingestellt, dass ihre Viskosität im Bereich von 50 mPas bis 100 Pa s, bevorzugt 100 mPas bis 10 Pa s, besonders bevorzugt 100 mPa·s bis 5 Pa s liegt. Die Viskosität wird bei 25°C mit einem Kegel-Platte-Viskosimeter bestimmt (Scherrate 100/s). Besonders bevorzugt weisen die erfindungsgemäßen Zusammensetzungen eine Viskosität < 10 Pa s und ganz besonders bevorzugt < 5 Pa s bei 25°C auf. Die Viskosität wird vorzugsweise mit einem Anton Paar Viskosimeter des Typs MCR 302 mit CP25-2 Konus-Platte-Messmittel und einem Messspalt von 53 µm in Rotation bei einer Scherrate von 100/s bestimmt. Aufgrund der geringen Viskosität sind die erfindungsgemäßen Zusammensetzungen besonders dazu geeignet, mit Hilfe von generativen Fertigungsverfahren, wie z.B. 3D-Druck oder Stereolithographie, verarbeitet zu werden. Die Verarbeitungstemperatur liegt vorzugsweise in einem Bereich von 10 bis 70°C, besonders bevorzugt 20 bis 30°C.

Erfindungsgemäß wurde überraschend gefunden, dass durch die Kombination von Bruchzähigkeitsmodifikatoren mit einem Übertragungsreagenz der Formel I eine deutliche Verbesserung der Bruchzähigkeit auch ohne die Verwendung von monofunktionellen Monomeren möglich ist. Dies gilt insbesondere für die Kombination mit den erfindungsgemäß bevorzugten Bruchzähigkeitsmodifikatoren. Auf diese Weise kann die Menge an monofunktionellen Monomeren reduziert oder ganz auf ihre Zugabe und die damit verbundenen Nachteile verzichtet werden. Die erfindungsgemäßen Zusammensetzungen zeichnen sich weiter dadurch aus, dass sie neben einer hohen Bruchzähigkeit und Brucharbeit gleichzeitig eine gute Biegefestigkeit und ein relativ hohes E-Modul aufweisen, gemessen nach Lagerung bei 37°C in Wasser, was oralen Bedingungen entspricht. Die Werkstoffe haben außerdem eine niedrige Viskosität. Besonders vorteilhaft ist darüber hinaus, dass die Dentalwerkstoffe auch nach der Härtung eine geringe Eigenfarbe haben.

Die erfindungsgemäßen Werkstoffe haben nach der Härtung eine Biegefestigkeit von größer 60 MPa, bevorzugt größer 70 MPa, besonders bevorzugt größer 75 MPa, einen Biegemodul von größer 1,50 GPa, bevorzugt größer 1,70 GPa, besonders bevorzugt größer 2,00 GPa, jeweils gemessen nach ISO 4049, und eine Brucharbeit FW größer 180 J/m², bevorzugt größer 200 J/m², besonders bevorzugt größer 210 J/m² auf, gemessen nach ISO 20795-1:2013. Werkstücke, die aus diesen Materialien hergestellt werden, halten somit in hohem Maße Verformungen ohne Bruch stand. Eine hohe Transparenz in Kombination mit einer hohen Bruchzähigkeit lassen sich mit Kern-Schale-Polymeren nicht erreichen.

Aufgrund der obigen Eigenschaften eignen sich erfindungsgemäßen Zusammensetzungen hervorragend als Dentalwerkstoffe und insbesondere zur Herstellung oder Reparatur von dentalen Formteilen. Die Erfindung betrifft auch die Verwendung der erfindungsgemäßen Zusammensetzungen als Dentalwerkstoff und insbesondere zur Herstellung oder Reparatur von dentalen Werkstücken, wie z.B. Dentalrestaurationen, Prothesen, Prothesenwerkstoffen, künstlichen Zähnen, Inlays, Onlays, Kronen, Brücken, Bohrschablonen, Einprobekörpern oder orthodontischen Vorrichtungen.

Außerdem betrifft die vorliegende Erfindung auch ein Verfahren zur Herstellung von dentalen Formteilen, insbesondere zur Herstellung der oben genannten dentalen Fortteilen, bei dem eine erfindungsgemäße Zusammensetzung mit Hilfe von Licht gehärtet wird, um das dentale Formteil zu ergeben. Die Erfindung betrifft auch dentale Formteile, insbesondere die oben genannten Formteile, die durch ein derartiges Verfahren erhältlich sind.

Die Herstellung oder Reparatur von dentalen Formteilen erfolgt insbesondere extraoral. Des Weiteren ist es bevorzugt, dass die Herstellung oder Reparatur von dentalen Formteilen durch ein generatives Verfahren, insbesondere mit Hilfe von 3D-Druck oder einem Lithographie-basierten Verfahren, wie z.B. der Stereolithographie, erfolgt.

Hierzu wird durch die direkte oder indirekte Digitalisierung des zu restaurierenden Zahns bzw. der zu restaurierenden Zähne am Computer ein virtuelles Abbild der Zahnsituation erstellt, dann anhand dieses Abbilds am Computer ein Modell der Dentalrestauration oder Prothese konstruiert und dieses dann durch generative stereolithographische Fertigung hergestellt. Nach Erstellung eines virtuellen Modells der herzustellenden Dentalrestauration oder Prothese wird die erfindungsgemäße Zusammensetzung durch selektive Lichteinstrahlung polymerisiert. Die Geometrie der Dentalrestauration oder Prothese kann schichtweise aufgebaut werden, indem nacheinander eine Vielzahl von dünnen Schichten mit gewünschtem Querschnitt polymerisiert wird. Nach dem schichtweisen Aufbau der Geometrie schließt sich vorzugsweise eine Reinigung des Werkstücks durch Behandlung mit einem geeigneten Lösungsmittel, wie z.B. einem Alkohol, wie Ethanol oder Isopropanol, einem Keton oder einem Ester, und eine Nachvergütung durch Bestrahlung des Werkstücks mit einer geeigneten Wellenlänge an, wie z.B. eine Bestrahlung mit einer Intensität von z.B. 25 mW/cm² bei 405 nm und gleichzeitig 130 mW bei 460 nm für 15 min, gegebenenfalls unter gleichzeitiger Erwärmung auf 50°C oder mehr, um den Restmonomergehalt weiter zu verringern und die mechanischen Eigenschaften zu verbessern.

Die Erfindung wird im Folgenden anhand von Beispielen näher erläutert.

### Ausführungsbeispiele

### Beispiel 1

### Herstellung des ABA-Blockcopolymers BC-1 (PCL(800)-b-PDMS(1600)-b-PCL(800)-Block-Copolymer)

Ein Gemisch aus Bisaminopropyl-terminierten Polydimethylsiloxan (PDMS von Gelest) mit einer Molmasse von 1600 g/mol (20,65 g) und ε-Caprolacton (CL: 21,10 g) wurde auf 80°C erhitzt. Nach 1 h wurde Zinn-bis(2-ethylhexanoat) (10 mg) zugegeben und die Badtemperatur wurde innerhalb von 30 min stufenweise auf 130°C erhöht. Das Reaktionsgemisch wurde weitere 22 h bei 130°C gerührt. Anschließend wurden flüchtige Komponenten am Feinvakuum abdestilliert. Man erhielt 40.47 g (97%) PCL(800)-b-PDMS(1600)-b-PCL(800)-Block-Copolymer BC-1 als wachsartigen, leicht bräunlichen Feststoff.

¹H-NMR (CDCl₃, 400 MHz): δ = 4.14 (t, 26H; *J* = 6.7 Hz; O-CH₂), 3.71 (t, 4H; *J* = 6.4 Hz; HO-CH₂), 3.30 (q, 4H; *J* = 6.4 Hz; N-CH₂), 2.39 (t, 26H; *J* = 7.5 Hz; C(O)-CH₂), 2.24 (t, 4H; *J* = 7.5 Hz; N-CH₂), 1.78-1.66 (m, 56H; CH₂), 1.53-1.36 (m, 30H; CH₂), 0.65-0.56 (m, 4H; Si-CH₂), 0.15 (s, 126H; Si-CH₃).

### Beispiel 2

### Herstellung und Härtung von Zusammensetzungen mit dem Urethandimethacrylat UDMA, dem Verdünner DEGDMA und dem Übertragungsreagenz TSMAE im Molverhältnis 7,2/1,8/1,0 und unterschiedlichen Mengen an ABA-Blockcopolymer BC-1

Die Monomerkomponenten Urethandimethacrylat UDMA (ein Additionsprodukt aus 2 mol 2-Hydroxyethylmethacrylat und 1 mol 2,2,4-Trimethylhexamethylen-1,6-diisocyanat) und Diethylenglycoldimethacrylat (DEGDMA) sowie das Übertragungsreagenz 2-Toluol-4-sulfonylmethyl)-acrylsäureethylester (TSMAE) wurden im Mol-Verhältnis 7,2 mol UDMA/1,8 mol DEGMA/1,0 mol TSMAE gemischt und mit 1,5 mol-% des Photoinitiators Diphenyl-(2,4,6-trimethylbenzoyl)phosphinoxid (TPO) versetzt. Die erhaltene Monomermischung wurde mit 3 Gew.-% (Monomerharz **R1**), 5 Gew.-% (Monomerharz **R2**) oder 10 Gew.-% (Monomerharz **R3**) des Blockcopolymeren BC-1 homogen gemischt. Dazu wurden die Komponenten mit einem Planetenmischer unter Rühren gelöst und bis zum Erreichen eines homogenen Gemisches weiter gerührt. Es wurde in allen Fällen vollkommen transparente Harze erhalten. Die Zusammensetzungen der Harzmischungen sind in Tabelle 1 angegeben.

**Tabelle 1: Zusammensetzung der Harze R1-R3 (Massen in Gew.-%)**

| **Monomerharz** | **UDMA^{b)}** | **DEGDMA^{d)}** | **TSMAE^{a)}** | **TPO^{C)}** | **BC-1** |
|---|---|---|---|---|---|
| **R1** | 79,22 | 10,24 | 6,32 | 1,22 | 3,00 |
| **R2** | 77,59 | 10,03 | 6,18 | 1,20 | 5,00 |
| **R3** | 73,51 | 9,49 | 5,86 | 1,14 | 10,00 |

| | | | | | |
|---|---|---|---|---|---|
| ^{a)} TSMAE: 2-(Toluol-4-sulfonylmethyl)-acrylsäureethylester ^{b)} UDMA: ein Additionsprodukt aus 2 mol 2-Hydroxyethylmethacrylat und 1 mol 2,2,4- Trimethylhexamethylen-1,6-diisocyanat ^{c)} Diphenyl-(2,4,6-trimethylbenzoyl)phosphinoxid (Photoinitiator) ^{d)} Diethylenglycoldimethacrylat | | | | | |

Mit den Harzen wurden in Metallformen Prüfkörper präpariert, die mit einer dentalen Lichtquelle (PrograPrint Cure, Fa. Ivoclar Vivadent AG, Schaan, Liechtenstein; Sofware: ProArt Print Splint, Jahr: 2020) beidseitig für 2 x 1,5 Minuten bestrahlt und damit ausgehärtet wurden.

Die weitere Bearbeitung und Einlagerung der Prüfkörper erfolgte gemäß den einschlägigen Vorgaben der nachfolgend genannten Vorschriften. Die Biegefestigkeit (BF) und der Biege-E-Modul (BM) wurden nach der ISO-Norm ISO-4049 (Dentistry - Polymerbased filling, restorative and luting materials) bestimmt. Dazu wurden die Prüfkörper vor der Messung entweder für 24 h bei Raumtemperatur (RT) trocken oder für 24 h bei 37°C in Wasser (H₂O) gelagert. Die Bestimmung der Bruchzähigkeit Kₘₐₓ und der Brucharbeit FW erfolgten gemäß ISO 20795-1:2013 (Zahnheilkunde - Kunststoffe - Teil 1: Prothesenkunststoffe). Die Ergebnisse der Messungen sind in Tabelle 2 zusammengestellt.

**Tabelle 2: Mechanische Eigenschaften der polymerisierten Harze R1-R3**

| **Harz** | BF 24 h, RT (MPa) | BF 24 h, 37°C H₂O (MPa) | BM 24 h, RT (MPa) | BM 24 h, 37°C H₂O (MPa) | Kₘₐₓ (MPa•m^{0,5}) | FW (J/m²) |
|---|---|---|---|---|---|---|
| **R1** | 102,9±1,8 | 102,6±1,3 | 2615±109 | 2574±98 | 1,35±0,05 | 181±7 |
| **R2** | 95,4±4,9 | 88,3±7,6 | 2537±66 | 2392±83 | 1,41±0,02 | 204±12 |
| **R3** | 81,1±1,5 | 77,5±5,4 | 2154±26 | 2033±40 | 1,50±0,04 | 289±13 |

Die Ergebnisse belegen, dass die Kombination von Übertragungsreagenz und Blockcopolymer auch ohne monofunktionelles Monomer Photopolymerisate mit guter Bruchzähigkeit und Biegefestigkeit ergeben.

### Beispiel 3

### Herstellung und Härtung von Zusammensetzungen mit dem Urethandimethacrylat UDMA, dem Verdünner DEGDMA und dem Übertragungsreagenz TSMAE im Molverhältnis 6,4/1,6/2,0 und unterschiedlichen Mengen an ABA-Blockcopolymer BC-1

Analog zu Beispiel 2 wurden die Monomerkomponenten UDMA und DEGDMA sowie das Übertragungsreagenz TSMAE im Mol-Verhältnis 6,4 mol UDMA/1,6 mol DEGMA/2,0 mol TSMAE gemischt und mit 1,5 mol-% des Photoinitiators TPO versetzt. Die erhaltene Monomermischung (Monomerharz **R4**) wurde mit 3 Gew.-% (Monomerharz **R5**), 5 Gew.-% (Monomerharz **R6**) oder 10 Gew.-% (Monomerharz **R7**) des Blockcopolymeren BC-1 homogen gemischt. Anschließend wurden Prüfkörper präpariert und die mechanischen Eigenschaften gemessen. Die Zusammensetzungen der Harzmischungen sind in Tabelle 3 und die Ergebnisse der Messungen in Tabelle 4 zusammengestellt.

**Tabelle 3: Zusammensetzung der Harze R4-R7 (Massen in Gew.-%)**

| **Monomerharz** | **UDMA^{b)}** | **DEGDMA^{d)}** | **TSMAE^{a)}** | **TPO^{c)}** | **BC-1** |
|---|---|---|---|---|---|
| **R4*)** | 75,57 | 9,68 | 13,43 | 1,31 | - |
| **R5** | 73,30 | 9,39 | 13,04 | 1,27 | 3,00 |
| **R6** | 71,79 | 9,20 | 12,76 | 1,25 | 5,00 |
| **R7** | 68,01 | 8,71 | 12,10 | 1,18 | 10,00 |

| | | | | | |
|---|---|---|---|---|---|
| *) Vergleichsbeispiel ^{a) - d)} wie Tabelle 1 | | | | | |

**Tabelle 4: Mechanische Eigenschaften der polymerisierten Harze R4-R7**

| **Harz** | BF 24 h, RT (MPa) | BF 24 h, 37°C H₂O (MPa) | BM 24 h, RT (MPa) | BM 24 h, 37°C H₂O (MPa) | Kₘₐₓ (MPa•m^{0,5}) | FW (J/m²) |
|---|---|---|---|---|---|---|
| **R4*)** | 100,3±6,3 | 102,8±6,9 | 2677±61 | 2642±107 | 0,99±0,05 | 95±9 |
| **R5** | 90,7±2,7 | 95,1±5,5 | 2479±90 | 2568±123 | 1,91±0,07 | 376±18 |
| **R6** | 95,5±6,3 | 93,4±6,8 | 2525±45 | 2356±84 | 1,71±0,06 | 339±31 |
| **R7** | 69,9±1,4 | 72,4±3,0 | 2050±26 | 2099±40 | 1,85±0,04 | 534±11 |

| | | | | | | |
|---|---|---|---|---|---|---|
| *) Vergleichsbeispiel | | | | | | |

Das Beispiel 3 zeigt im Vergleich mit Beispiel 2, dass die Bruchzähigkeit, d.h. Kmax und die Brucharbeit, der Photopolymerisate mit steigender Konzentration des Übertragungsreagenzes sehr deutlich zunehmen ohne dass eine wesentliche Reduktion der Biegefestigkeit bzw. des Biegemoduls auftritt. Die Ergebnisse belegen, dass durch den Einsatz von Übertragungsreagenzien in Kombination mit Blockcopolymeren auch ohne monofunktionelle Monomere Photopolymerisate mit guter Bruchzähigkeit erhalten werden können.

### Beispiel 4

### Herstellung und Härtung von Zusammensetzungen mit dem Urethandimethacrylat UDMA, dem Verdünner DEGDMA und dem Übertragungsreagenz TSMAE im Molverhältnis 5,6/1,4/3,0 und unterschiedlichen Mengen an ABA-Blockcopolymer BC-1

Analog zu Beispiel 2 wurden die Monomerkomponenten UDMA und DEGDMA sowie das Übertragungsreagenz TSMAE im Mol-Verhältnis 5,6 mol UDMA/1,4 mol DEGMA/3,0 mol TSMAE gemischt und mit 1,5 mol-% des Photoinitiators TPO versetzt. Die erhaltene Monomermischung wurde mit 3 Gew.-% (Monomerharz **R8**), 5 Gew.-% (Monomerharz **R9**) oder 10 Gew.-% (Monomerharz **R10**) des Blockcopolymeren BC-1 homogen gemischt. Anschließen wurden Prüfkörper präpariert und die mechanischen Eigenschaften gemessen. Die Zusammensetzungen der Harzmischungen sind in Tabelle 5 und die Ergebnisse der Messungen in Tabelle 6 zusammengestellt.

**Tabelle 5: Zusammensetzung der Harze R8-R10 (Massen in Gew.-%)**

| **Monomerharz** | **UDMA^{b)}** | **DEGDMA^{d)}** | **TSMAE^{a)}** | **TPO^{c)}** | **BC-1** |
|---|---|---|---|---|---|
| **R8** | 66,69 | 8,61 | 20,38 | 1,32 | 3,00 |
| **R9** | 65,31 | 8,44 | 19,96 | 1,29 | 5,00 |
| **R10** | 61,88 | 7,99 | 18,90 | 1,23 | 10,00 |

| | | | | | |
|---|---|---|---|---|---|
| ^{a) - d)} wie Tabelle 1 | | | | | |

**Tabelle 6: Mechanische Eigenschaften der polymerisierten Harze R8-R10**

| **Harz** | BF 24 h, RT (MPa) | BF 24 h, 37°C H₂O (MPa) | BM 24 h, RT (MPa) | BM 24 h, 37°C H₂O (MPa) | Kₘₐₓ (MPa•m^{0,5}) | FW (J/m²) |
|---|---|---|---|---|---|---|
| **R8** | 83,6±0,9 | 79,3±2,3 | 2486±59 | 2295±64 | 2,05±0,07 | 484±32 |
| **R9** | 77,7±4,4 | 72,9±1,3 | 2323±35 | 2120±99 | 2,21±0,06 | 617±39 |
| **R10** | 65,6±2,4 | 59,4±2,3 | 2071±91 | 1796±75 | 2,06±0,04 | 837±22 |

Das Beispiel 4 zeigt im Vergleich mit den Beispielen 2 und 3, dass durch eine Erhöhung der Konzentration an Übertragungsreagenz die Bruchzähigkeit der Photopolymerisate weiter gesteigert werden kann.

### Beispiel 5:

### Herstellung und Härtung von Zusammensetzungen ohne Übertragungsreagenz (Referenzbeispiel)

Zusätzlich zu den Beispielen **2** bis **4** wurden 2 Vergleichsbespiele durchgeführt, bei denen a) das Harz **R11** - analog **R4** - nur aus Urethandimethacrylat UDMA und dem Verdünner DEGDMA im Molverhältnis 8/2 **ohne** Übertragungsreagenz TSMAE und **ohne** Blockcopolymer untersucht wurde und b) ein Harz **R12** aus Urethandimethacrylat UDMA und dem Verdünner DEGDMA im Molverhältnis 8/2 **ohne** Übertragungsreagenz TSMAE aber **mit** Blockcopolymer BC-1 (10 Ge.-%) untersucht wurde. Die Harze **R11** und **R12** enthalten ebenfalls 1,5 Mol-% des Photoinitiators TPO und wurden analog zu den Beispielen **2** bis **4** als homogene Mischungen hergestellt, Prüfkörper präpariert und die mechanischen Eigenschaften gemessen. Die Zusammensetzungen der Harzmischungen sind in Tabelle 7 und die Ergebnisse der Messungen in Tabelle 8 zusammengestellt.

**Tabelle 6: Zusammensetzung der Harze R11-R12 (Massen in Gew.-%)**

| **Monomerharz** | **UDMA^{b)}** | **DEGDMA^{d)}** | **TSMAE^{a)}** | **TPO^{c)}** | **BC-1** |
|---|---|---|---|---|---|
| **R11*)** | 87,52 | 11,26 | - | 1,22 | - |
| **R12*)** | 78,77 | 10,14 | - | 1,09 | 10,00 |

| | | | | | |
|---|---|---|---|---|---|
| * Vergleichsbeispiel ^{a) - d)} wie Tabelle 1 | | | | | |

**Tabelle 8: Mechanische Eigenschaften der polymerisierten Harze R11-R12**

| **Harz** | BF 24 h, RT (MPa) | BF 24 h, 37°C H₂O (MPa) | BM 24 h, RT (MPa) | BM 24 h, 37°C H₂O (MPa) | Kₘₐₓ (MPa•m^{0,5}) | FW (J/m²) |
|---|---|---|---|---|---|---|
| **R11** | 119,3±3,3 | 124,5±5,4 | 2871±58 | 2857±75 | 1,18±0,06 | 132±12 |
| **R12** | 95,9±2,1 | 92,2±2,3 | 2392±66 | 2164±27 | 1,21±0,04 | 196±15 |

Der Vergleich der Referenzbeispiele **R4, R11** und **R12** mit den erfindungsgemäßen Beispielen **R1** bis **R3** und **R5** bis **R10**) belegt, dass eine gute Bruchzähigkeit (Kₘₐₓ) und eine hohe Brucharbeit (FW) nur durch die Kombination von Übertragungsreagenz und Blockcopolymer erreicht werden kann.

## Patentansprüche

1. Radikalisch polymerisierbare Zusammensetzung, die mindestens ein urethangruppenhaltiges (Meth)acrylatmonomer, mindestens einen Bruchzähigkeitsmodifikator und mindestens ein Übertragungsreagenz enthält, **dadurch gekennzeichnet dass** das Übertragungsreagenz eine Verbindung der allgemeinen Formel I ist: 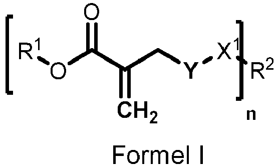 in der die Variablen die folgenden Bedeutungen haben:
R¹ Wasserstoff, ein aliphatischer linearer oder verzweigter C₁-C₁₅-Alkyl-Rest, der durch ein oder mehrere, vorzugsweise 1 bis 3, O oder S unterbrochen und/oder durch eine oder mehrere, vorzugsweise 1, C₁-C₅-Alkylgruppen und/oder OH substituiert oder unsubstituiert sein kann, Benzyl oder Phenyl, die durch eine oder mehrere, vorzugsweise eine, C₁-C₃-Alkylgruppen substituiert oder unsubstituiert sein können;
Y S oder SO₂;
X¹ entfällt oder ein aliphatischer linearer oder verzweigter C₁-C₁₅-Alkyl-Rest, der durch eine Estergruppe (-CO-O-), Urethangruppe (-O-CO-NH-), ein oder mehrere, vorzugsweise 1 bis 3, O oder S unterbrochen und/oder durch eine oder mehrere, vorzugsweise 1, C₁-C₅-Alkylgruppen und/oder OH substituiert oder unsubstituiert sein kann;
R² ein aliphatischer linearer oder verzweigter C₁-C₂₀-Alkyl-Rest, der durch eine Estergruppe (-CO-O-), Urethangruppe (-O-CO-NH-), ein oder mehrere, vorzugsweise 1 bis 3, O oder S unterbrochen und/oder durch 1 bis 3 OH Gruppen substituiert oder unsubstituiert sein kann, ein cycloaliphatischer C₅₋C₂₀-Rest, ein aromatischer C₆-C₂₀-Rest oder ein Isocyanursäurerest, die jeweils durch eine oder mehrere, vorzugsweise eine, C₁-Cs-Alkylgruppen substituiert oder unsubstituiert sein können;
n 1, 2 oder 3.

2. Radikalisch polymerisierbare Zusammensetzung nach Anspruch 1, wobei die Variablen der Formel I die folgenden Bedeutungen haben:
R¹ aliphatischer linearer oder verzweigter C₁-C₁₀-Alkyl-Rest, der durch 1 bis 3 O oder S unterbrochen und/oder durch 1 bis 3 Methylgruppen substituiert oder unsubstituiert sein kann;
Y S oder SO₂;
X¹ entfällt oder ein aliphatischer linearer oder verzweigter C₁-C₁₀-Alkyl-Rest, der durch eine Estergruppe (-CO-O-), Urethangruppe (-O-CO-NH-), 1 bis 3 O oder S unterbrochen und/oder durch 1 bis 3 Methylgruppen substituiert oder unsubstituiert sein kann;
R² ein aliphatischer linearer oder verzweigter C₁-C₁₆-Alkyl-Rest, der durch eine Estergruppe (-CO-O-), Urethangruppe (-O-CO-NH-), 1 bis 3 O oder S unterbrochen und/oder durch eine OH Gruppe substituiert sein kann, oder ein aromatischer C₆-C₂₀ Rest, der durch eine Methylgruppe substituiert oder unsubstituiert ist;
n 1 oder 2.

3. Radikalisch polymerisierbare Zusammensetzung nach Anspruch 2, wobei die Variablen der Formel I die folgenden Bedeutungen haben:
R¹ ein aliphatischer linearer oder verzweigter C₁-C₆-Alkyl-Rest, der durch ein oder mehrere, vorzugsweise 1 bis 2, O oder S unterbrochen sein kann, vorzugsweise ein linearer C₁-C₃-Alkylrest;
Y S oder SO₂, vorzugsweise SO₂;
X¹ entfällt;
R² ein aliphatischer linearer oder verzweigter C₁-C₁₂-Alkyl-Rest, der durch eine Estergruppe (-CO-O-), Urethangruppe (-O-CONH-), 1 bis 2 O oder S unterbrochen und/oder durch eine OH Gruppe substituiert oder unsubstituiert sein kann, oder ein aromatischer C₆-C₂₀ Rest, vorzugsweise eine methylsubstituierte Phenylgruppe;
n 1.

4. Radikalisch polymerisierbare Zusammensetzung nach einem der Ansprüche 1 bis 3, wobei das urethangruppenhaltige (Meth)acrylatmonomer ein Monomer oder eine Mischung von Monomeren ist, das oder die einen Urethangruppengehalt von 0,01 bis 5 mmol/g, vorzugsweise 0,1 bis 4,5 mmol/g und besonders bevorzugt 0,5 bis 4,0 mmol/g aufweist/aufweisen.

5. Radikalisch polymerisierbare Zusammensetzung nach Anspruch 4, wobei das urethangruppenhaltige (Meth)acrylatmonomer ein Urethandi(meth)acrylat ist, vorzugsweise ein Urethandi(meth)acrylat mit einer Molmasse von kleiner 2000 g/mol, und besonders bevorzugt ein Urethandi(meth)acrylat mit einer Molmasse von kleiner 2000 g/mol, das 1 bis 4 Urethangruppen enthält.

6. Radikalisch polymerisierbare Zusammensetzung nach einem der Ansprüche 1 bis 5, die als Bruchzähigkeitsmodifikator ein Blockcopolymer und/oder Polymerpartikel mit Kern-Schale-Struktur enthält.

7. Radikalisch polymerisierbare Zusammensetzung nach Anspruch 6, die als Blockcopolymer ein AB-Diblock- oder ein ABA-Triblockcopolymer enthält, wobei
der A-Block ein Oligomer ist, das aus einem oder mehreren der folgenden Monomere aufgebaut ist: cyclische, aliphatische Ester oder Ether, Arylenoxid, Alkylenoxid, radikalisch polymerisierbare Monomere, und
der B-Block ein Polysiloxan- und/oder ein Polyvinyl- und/oder ein Polyalken- und/oder ein Polydien-Oligomer und/oder ein hydriertes Polydien-Oligomer ist.

8. Radikalisch polymerisierbare Zusammensetzung nach Anspruch 7, wobei
der A-Block ist ein Polymerisat von Caprolacton, 2,6-Dialkyl-1,4-phenylenoxid und insbesondere von 2,6-Dimethyl-1,4-phenylenoxid, Ethylenoxid, Propylenoxid oder (Meth)acrylaten ist, und
der B-Block ist ein Polydien-Oligomer, ein hydriertes Polydien-Oligomer, Polyvinyalkanoat-Oligomer oder ein Polysiloxan-Oligomer gemäß der Formel -O-(SiR¹²₂-O)_{P}- ist, in der
R¹² eine lineare C₁-C₂₀-Alkyl-, verzweigte C₃-C₁₂-Alkyl- oder C₆-C₂₀-Arylgruppe ist, wobei die einzelnen R¹²-Reste gleich oder verschieden sein können, und
p eine Zahl von 3 bis 100, bevorzugt eine Zahl von 10 bis 50 ist.

9. Radikalisch polymerisierbare Zusammensetzung nach einem der Ansprüche 1 bis 8 die
(a) 1 bis 30 Gew.-% mindestens eines Übertragungsreagenzes der Formel I,
(b) 1 bis 97,9 Gew.-% mindestens eines urethangruppenhaltigen (Meth)acrylatmonomers,
(c) 0 bis 20 Gew.-% eines oder mehrerer Mono(meth)acrylate,
(d) 0 bis 50 Gew.-% eines oder mehrerer Di(meth)acrylate ohne Urethangruppen,
(e) 1 bis 10 Gew.-% mindestens eines Bruchzähigkeitsmodifikators,
(f) ggf. 10 bis 5000 ppm eines oder mehrerer Inhibitoren,
(g) 0,1 bis 5,0 Gew.-% mindestens eines Photoinitiators für die radikalische Polymerisation,
(h) 0 bis 30 Gew.-% eines oder mehrerer anorganischer oder organischer Füllstoffe,
(i) 0 bis 30 Gew.-% eines oder mehrerer Additive enthält,
wobei sich alle Prozentangaben auf die Gesamtmasse der Zusammensetzung beziehen.

10. Radikalisch polymerisierbare Zusammensetzung nach Anspruch 9, die
(a) 2 bis 25 Gew.-% und bevorzugt 3 bis 20 Gew.-% mindestens eines Übertragungsreagenzes der Formel I,
(b) 20 bis 95,84 Gew.-% und bevorzugt 30 bis 93,78 Gew.-% mindestens eines Urethandi(meth)acrylats,
(c) 0 bis 10 Gew.-% und bevorzugt 0 Gew.-% Mono(meth)acrylate,
(d) 0 bis 40 Gew.-% und bevorzugt 0 bis 30 Gew.-% eines oder mehrerer Di(meth)acrylate ohne Urethangruppen,
(e) 2 bis 8 Gew.-% und bevorzugt 3 bis 6 Gew.-% mindestens eines Bruchzähigkeitsmodifikators,
(f) 100 bis 4000 ppm und bevorzugt 150 bis 1500 ppm mindestens eines phenolischen Inhibitors,
(g) 0,15 bis 4,0 Gew.-% und bevorzugt 0,2 bis 3,0 Gew.-% mindestens eines Photoinitiators für die radikalische Polymerisation,
(h) 0 bis 20 Gew.-% und bevorzugt 0 bis 10 Gew.-% eines oder mehrerer anorganischer oder organischer Füllstoffe,
(i) 0 bis 25 Gew.-% und bevorzugt 0 bis 20 Gew.-% eines oder mehrerer Additive enthält, jeweils bezogen auf die Gesamtmasse der Zusammensetzung.

11. Radikalisch polymerisierbare Zusammensetzung nach einem der Ansprüche 1 bis 10, die keine monofunktionellen Monomere und/oder keine Polymerpartikel mit Kern- Schale-Struktur enthält.

12. Verwendung einer Zusammensetzung gemäß einem der Ansprüche 1 bis 11 als Dentalwerkstoff.

13. Verwendung nach Anspruch 12 zur Herstellung dentaler Formteile durch ein generatives Verfahren.

14. Verwendung einer Kombination eines Bruchzähigkeitsmodifikators mit einem Übertragungsreagenz der Formel I zur Verbesserung der Bruchzähigkeit radikalisch polymerisierbarer Zusammensetzungen.

15. Verfahren zur Herstellung dentaler Formkörper, bei dem man
(i) durch die direkte oder indirekte Digitalisierung des zu restaurierenden Zahns bzw. der zu restaurierenden Zähne am Computer ein virtuelles Abbild der Zahnsituation erstellt,
(ii) man dann anhand dieses Abbilds am Computer ein Modell der Dentalrestauration oder Prothese konstruiert,
(iii) man durch schichtweise Polymerisation einer Zusammensetzung gemäß einem der Ansprüche 1 bis 11 mittels selektiver Lichteinstrahlung die Dentalrestauration oder Prothese schichtweise aufbaut.
